(19) Europäisches Patentamt / European Patent Office / Office européen des brevets

(11) **EP 1 462 605 B1**

(12) **FASCICULE DE BREVET EUROPEEN**

(45) Date de publication et mention
de la délivrance du brevet:
**03.06.2009 Bulletin 2009/23**

(51) Int Cl.:
*E21B 49/00* (2006.01)    *E21B 47/00* (2006.01)
*G01N 33/28* (2006.01)

(21) Numéro de dépôt: **04290745.1**

(22) Date de dépôt: **18.03.2004**

(54) **Méthode de pseudoisation et d'éclatement pour décrire des fluides hydrocarbones**

Verfahren zur Modellieren eine Kohlenwasserstoffmischung durch Unterteilung in Pseudokomponenten

Method of modelling a hydrocarbon mixture by subdividing it in pseudocomponents

(84) Etats contractants désignés:
**FR GB IT NL**

(30) Priorité: **28.03.2003 FR 0303908**

(43) Date de publication de la demande:
**29.09.2004 Bulletin 2004/40**

(73) Titulaire: **Institut Français du Pétrole**
**92852 Rueil-Malmaison Cedex (FR)**

(72) Inventeur: **Barroux, Claire**
**92370 Chaville (FR)**

(56) Documents cités:
**US-A- 5 710 726**    **US-A- 6 128 579**

- **JOERGENSEN M ET AL: "OPTIMIZATION OF PSEUDO-COMPONENT SELECTION FOR CMPOSITIONAL STUDIES OF RESERVOIR FLUIDS" SPE ANNUAL TECHNICAL CONFERENCE AND EXHIBITION, XX, XX, no. SPE 30789, 1995, pages 917-927, XP001080675**
- **NEWLEY T M J ET AL: "PSEUDOCOMPONENT SELECTION FOR COMPOSITIONAL SIMULATION" SPE ANNUAL TECHNICAL CONFERENCE AND EXHIBITION, XX, XX, no. SPE 19638, 1989, pages 117-127, XP001080674**
- **LOLLEY C S ET AL: "Minimum pseudocomponent requirements for compositional thermal simulation of heavy oil" PROCEEDINGS OF THE 1998 11TH SYMPOSIUM ON IMPROVED OIL RECOVERY. PART 1 (OF 2); TULSA, OK, USA APR 19-22 1998, vol. 1, no. SPE 39640, 1998, pages 383-400, XP002264507 Richardson, TX, USA**
- **BUCHWALTER J L ET AL: "New simplified compositional simulator" PROCEEDINGS OF THE ROCKY MOUNTAIN REGIONAL MEETING/LOW PERMEABILITY RESERVOIRS SYMPOSIUM AND EXHIBITION;DENVER, CO, USA APR 26-28 1993, no. SPE 25858, 1993, pages 177-188, XP002264508 Richardson, TX, USA**

EP 1 462 605 B1

## Description

La présente invention concerne une méthode dite de pseudoïsation, groupement ou « lumping » pour décrire un fluide formé d'un mélange de multiples constituants par un nombre réduit de constituants (au moins trois), et une méthode permettant l'opération inverse, méthode dite de dégroupement ou « delumping », c'est-à-dire une méthode permettant l'obtention de la composition détaillée du fluide à partir de la description par un nombre réduit de constituants.

La présente invention concerne donc une méthode de modélisation:

- permettant d'estimer les propriétés ou le comportement des phases liquide et/ou vapeur de mélanges de multiples composants, tels que rencontrés in situ dans les réservoirs de pétrole ou de gaz ou en surface lors de l'exploitation de ces mêmes gisements, à l'aide d'une représentation à nombre réduit de pseudo-composants,

- et offrant la possibilité de prédire en fonction du temps la composition détaillée de fluides produits au cours de la production.

L'invention est particulièrement utile pour accélérer les calculs lors de la simulation de l'exploitation de gisements souterrains d'hydrocarbures.

Un tel modèle permet à l'ingénieur de gisement de réduire les temps de calcul des simulations de comportement de gisement soumis à une exploitation, tout en conservant une bonne modélisation des mécanismes d'échanges entre phases hydrocarbure, et d'établir des profils compositionnels détaillés, ceux-ci étant nécessaires en particulier au dimensionnement prévisionnel et à la gestion d'installations de surface, telles que séparateurs, unités de traitement, lignes de transport, etc., donc utiles en ingénierie de surface et de procédés.

## ETAT DE LA TECHNIQUE

La modélisation des écoulements dans un réservoir pétrolier ou dans un stockage sous-terrain est fondée essentiellement sur l'application au réservoir préalablement maillé (ou à une portion de celui-ci) de la loi de Darcy bien connue décrivant l'écoulement de fluides en milieux poreux, de lois de bilans de matière dans chaque unité de volume, des relations thermodynamiques régissant l'évolution des propriétés de phase des fluides tels que la viscosité, la masse volumique, sur des conditions initiales, sur des conditions aux limites de fermeture de la structure, et sur des conditions aux puits.

Le modèle dit « Black Oil », désigné ci-après en abrégé par B.O., est l'un des plus utilisés en simulation pétrolière. Il permet de décrire un écoulement tridimensionnel et triphasique (eau-huile-gaz) compressible. Les effluents pétroliers intervenant dans ce modèle sont décrits généralement par un constituant eau, et deux constituants pour le fluide de gisement, le mot de constituant recouvrant ici la notion de composant (comme H2O pour l'eau) et la notion de pseudo-composant (regroupement de composants). Les constituants intervenant dans ce modèle sont au nombre de trois : un constituant eau (W), un constituant hydrocarbure lourd (H), et un constituant hydrocarbure léger (V). Dans un modèle de type B.O. dit « strict », le constituant (W) n'est présent que dans la phase eau, le constituant (H) n'est présent que dans la phase liquide hydrocarbure (appelée huile ou condensat), le constituant (V) se partage entre les phases hydrocarbure liquide et vapeur (phase appelée gaz). Un modèle B.O. dit « étendu » diffère d'un modèle B.O. « strict » en ce que le constituant (H) se partage entre les phases hydrocarbure liquide et vapeur. Mais si l'usage des modèles B.O. est applicable à un grand nombre de cas industriels, il n'en reste pas moins à déconseiller dans certains cas, en particulier des cas de gisements de gaz à condensats soumis à une injection de gaz sec.

Un autre modèle bien connu de simulation dit « compositionnel » est aussi utilisé, où les fluides hydrocarbure sont représentés par un nombre plus élevé de constituants, au moins trois, souvent davantage, le constituant eau n'étant présent que dans la phase aqueuse. La modélisation des flux de ces fluides mieux détaillés se traduit par des temps de calcul d'autant plus importants que le nombre de constituants est élevé.

Pour que les calculs de modélisation puissent être menés dans un délai raisonnable, les fluides en place sont décrits comme constitués d'un nombre de composants ou pseudo-composants beaucoup plus réduit que le nombre réel de composants. Le passage d'une représentation détaillée des fluides à une représentation à nombre moindre de constituants s'effectue par des opérations dites de « lumping » (groupement ou regroupement) ou de « pseudoïsation ». Dans ce qui suit, sauf indication contraire, le terme, de « pseudoïsation » sera employé pour toute méthode permettant de réduire le nombre de constituants.

Différentes méthodes de pseudoïsation ont déjà été proposées pour sélectionner, définir les pseudo-composants, l'ingénieur se trouvant souvent à devoir trouver un compromis entre la précision, et le temps (et le coût) des calculs. Pour la simulation de l'exploitation de gisements de gaz à condensat soumis à une injection de gaz sec, ce sont des représentations à quelque 6 ou 8 constituants qui sont en général utilisées, ce qui se traduit par des temps de calcul d'autant plus importants qu'il est souvent nécessaire de réduire la taille des mailles pour limiter l'erreur numérique et par conséquent d'augmenter le nombre de mailles. Des efforts considérables sont donc encore aujourd'hui consacrés à l'élaboration de méthodes de pseudoïsation à l'usage de l'industrie, qui permettraient de réduire encore le nombre de constituants, tout en modélisant le comportement des fluides avec précision, et en offrant la possibilité de recouvrer une information compositionnelle détaillée. Les opérations qui permettent de prédire quels auraient été les résultats d'une simulation de réservoir si l'on avait utilisé une modélisation finement détaillée (où les fluides sont représentés par un nombre plus élevé de composants) sont connues des gens de l'art sous l'appellation de « delumping », que l'on désignera par la suite aussi par « éclatement ».

Par la demande de brevet WO 00/37898, on connaît une méthode de pseudoïsation, applicable à des simulations compositionnelles, basée sur la sélection d'un nombre de composants de base 'dominants' égal au nombre de pseudo-composants que l'on veut obtenir à l'issue de la procédure. Dans cette méthode, les composants non dominants sont représentés dans tous les pseudo-composants, et un composant dominant particulier n'est représenté que dans un seul pseudo-composant. La transformation mathématique à la base de la méthode de pseudoïsation permet, par transformation inverse, de recouvrer l'information compositionnelle détaillée. La composition d'un pseudo-composant pris en particulier peut faire apparaître des fractions molaires négatives de constituants de base, comme dans l'exemple donné table E7 de la demande de brevet en référence. On conçoit qu'une telle représentation perd un certain sens physique dès lors que l'on considère isolément un pseudo-composant particulier. Cela semble pouvoir poser un problème de robustesse, lorsque, comme il arrive en pratique dans les cas d'injection de gaz, un résultat 'local' de la simulation, par exemple dans certaines mailles, indique la disparition de l'un ou plusieurs des constituants utilisés dans la simulation dynamique. Par ailleurs, la mise en oeuvre de l'invention est décrite comme nécessitant de nombreux calculs itératifs et un large espace mémoire.

Par des publications antérieures, on connaît des méthodes de pseudoïsation, applicables également à des simulations compositionnelles, où chaque pseudo-composant est formé en regroupant plusieurs constituants de base, un constituant de base particulier ne se trouvant représenté que dans un seul pseudo-composant. Le regroupement peut être effectué selon un choix de critères fixés a priori, tels que ceux cités dans la demande de brevet mentionné ci-dessus, ou par une procédure d'optimisation, par exemple telle que proposée par K. Liu dans le papier « Reduce the Number of Components for Compositional Reservoir Simulation », SPE 66363, présenté à la SPE Reservoir Simulation Symposium, Houston, Texas, 11-14 Février 2001.

Un papier, bien connu des gens de l'art, écrit par D.E. Kenyon et G. Alda Behie « Third SPE Comparative Solution Project: Gas Cycling of Retrograde Condensate Reservoirs », SPE 12278, Journal of Petroleum Technology, Août 1987, illustre une situation non exceptionnelle : il arrive de constater, pour un même cas d'étude, une grande disparité dans les résultats de simulations compositionnelles lorsqu'elles sont réalisées avec des représentations de fluides différentes, et qui plus est, avec des logiciels de simulation différents. Dans ce papier, le nombre des constituants varie de 5 à 16, et la disparité des résultats est en partie imputable aux divers choix de représentations compositionnelles des fluides: la tendance générale observée est que plus le nombre de constituants est faible, plus la saturation en liquide hydrocarbure (appelé huile ou condensat) dans une maille particulière peut être sous-estimée, et plus la récupération d'huile en surface est alors surestimée.

Pour recouvrer l'information compositionnelle détaillée au cours d'une simulation compositionnelle de réservoir, on peut utiliser des méthodes de delumping telles que celle exposée dans brevet WO 99/42937 et aussi dans le papier de C. Leibovici et J. Barker « A Method for Delumping the Results of a Compositional Reservoir Simulation » SPE 49068, présenté à la SPE Annual Technical Conference and Exhibition New-Orleans, 27-30 Septembre 1998. La méthode permet de prévoir l'évolution de la composition détaillée au cours du temps à partir des calculs, en particulier de coefficients d'équilibre, effectués dans une simulation de type compositionnel de fluides décrits par un certain nombre réduit de pseudo-composants, le nombre de composants étant au moins de trois.

Une représentation de type Black-Oil peut être considérée comme issue d'une opération de pseudoïsation particulière fournissant deux pseudo-composants. La composition détaillée de chacun des pseudo-composants, qui n'est pas utile à la construction de la représentation, n'est pas connue a priori, sans que ceci soit un obstacle rédhibitoire au recouvrement d'une information compositionnelle détaillée, par une opération de delumping. Ainsi, par la demande de brevet FR 00/09008 publiée comme FR 28 11 430, on connaît une méthode qui permet de prévoir l'évolution de la composition détaillée au cours du temps à partir des calculs effectués dans une simulation dynamique de type Black-Oil.

Le principe de l'étape d'éclatement (ou delumping) les demandes de brevets WO 99/42937 et FR 00/09008 publiée comme FR 28 11 430 est de déterminer, - à partir des calculs effectués pendant la simulation, avec la représentation thermodynamique regroupée (compositionnelle ou BO), dans chaque maille et à chaque pas de temps -, le coefficient $\Delta D_0$ et les n coefficients $\Delta D_p$ (soit n+1 coefficients, n étant le nombre de paramètres de l'équation d'état) d'une équation générale connue, publiée antérieurement dans un papier de C.F. Leibovici, E.H. Stenby, K. Knudsen, « A Consistent Procedure for Pseudo-Component Delumping », Fluid Phase Equilibria, 1996, 117, 225-232. :

$$(1) \qquad Ln(K_i) = \Delta D_0 + \sum_{p=1}^{n} \Delta D_p \, \Pi_{pi}$$

où $K_i$ est la constante d'équilibre du constituant i et les $\Pi_{pi}$ sont des paramètres fixes de caractérisation du constituant i dans l'équation d'état pour une représentation thermodynamique donnée.

Une fois le coefficient $\Delta D_0$ et les n coefficients $\Delta D_p$ déterminés, on les utilise pour calculer les constantes d'équilibre des constituants de la représentation thermodynamique détaillée ($N_{rb}$ composants) en appliquant l'équation (1) aux $N_{rb}$ composants avec leurs propres paramètres fixes de caractérisation dans la représentation thermodynamique détaillée.

L'un des intérêts de cette méthode est que, dans l'étape d'éclatement (« delumping »), il n'est pas nécessaire de résoudre les $N_{rb}$ équations d'équilibre associées à l'équation d'état (équations qui traduisent l'égalité des fugacités de chaque constituant dans chaque phase) aux différents pas de temps de la simulation dynamique des écoulements, ce qui constitue une économie de temps de calcul.

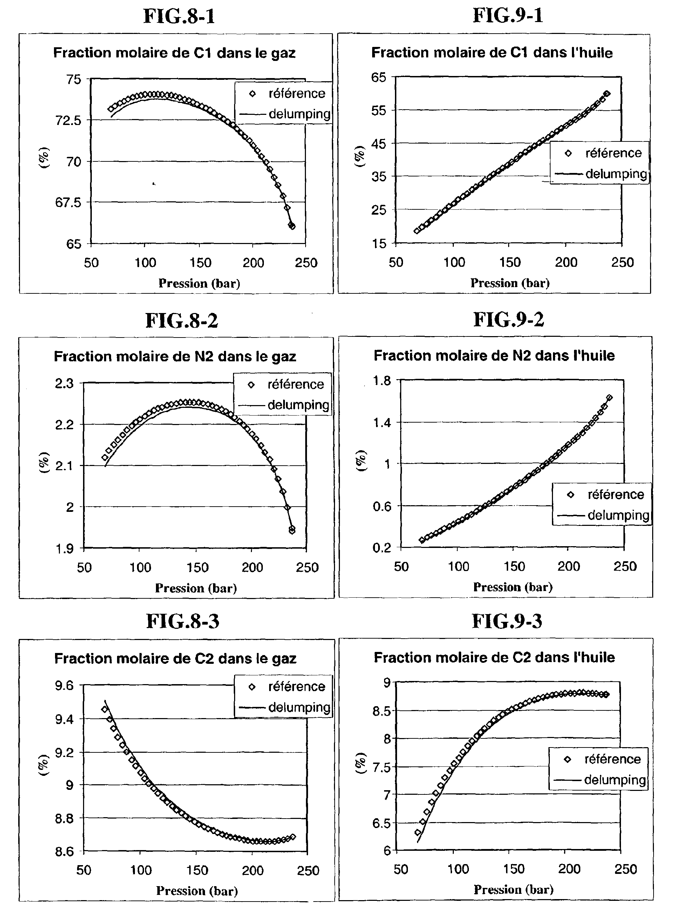

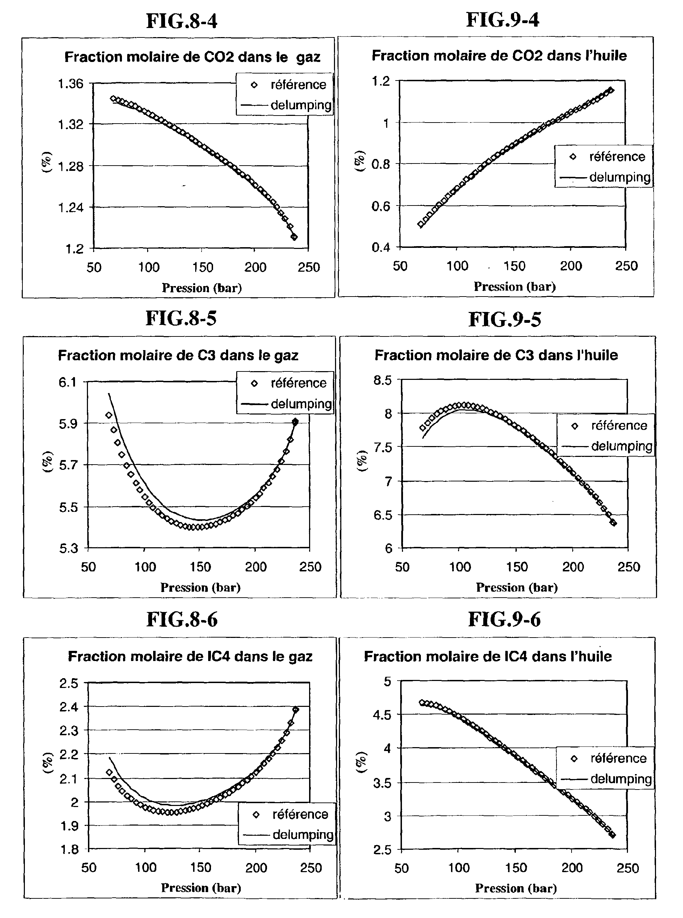

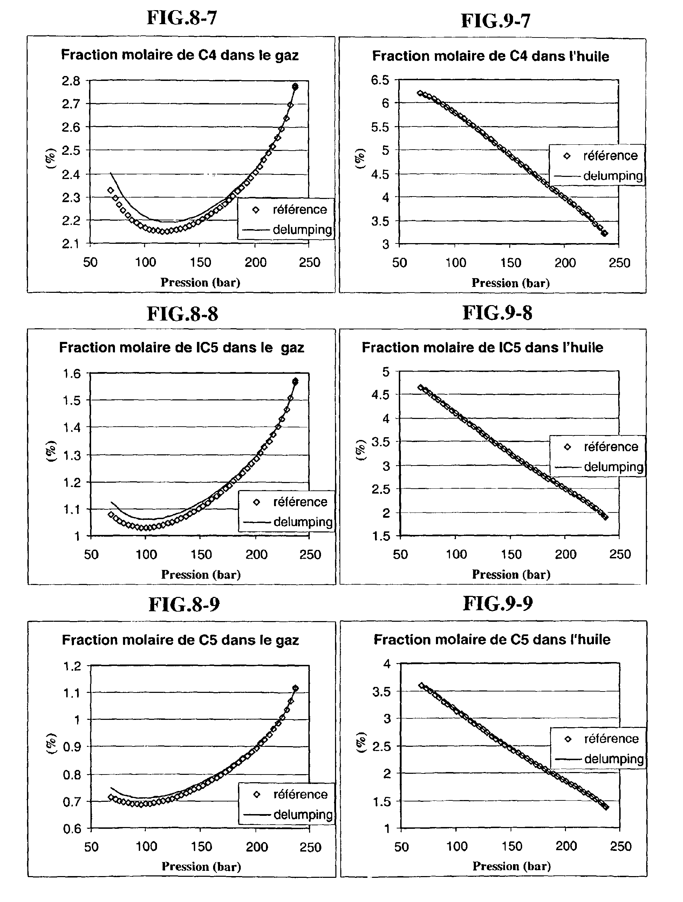

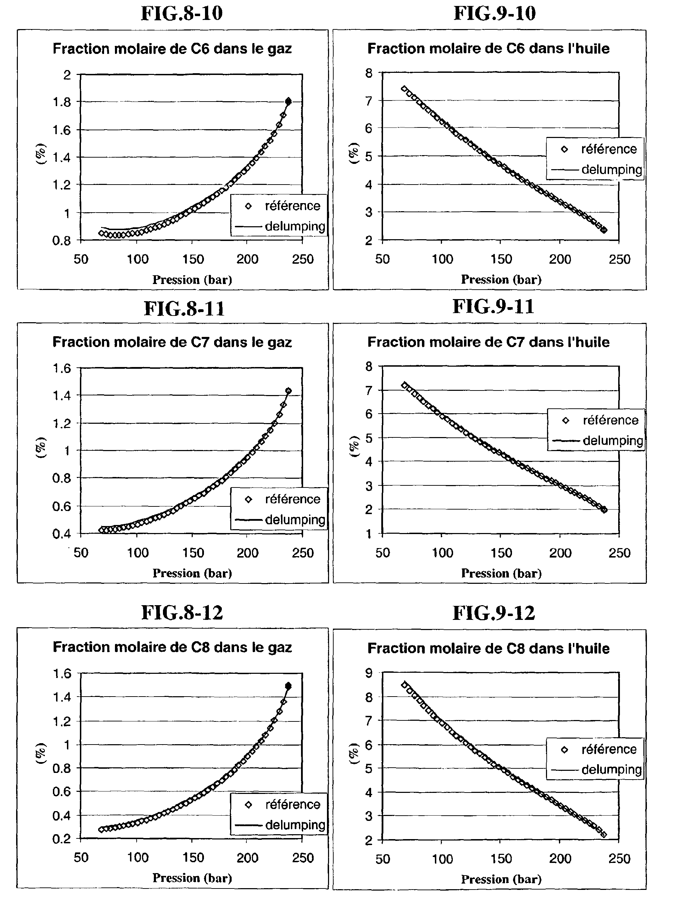

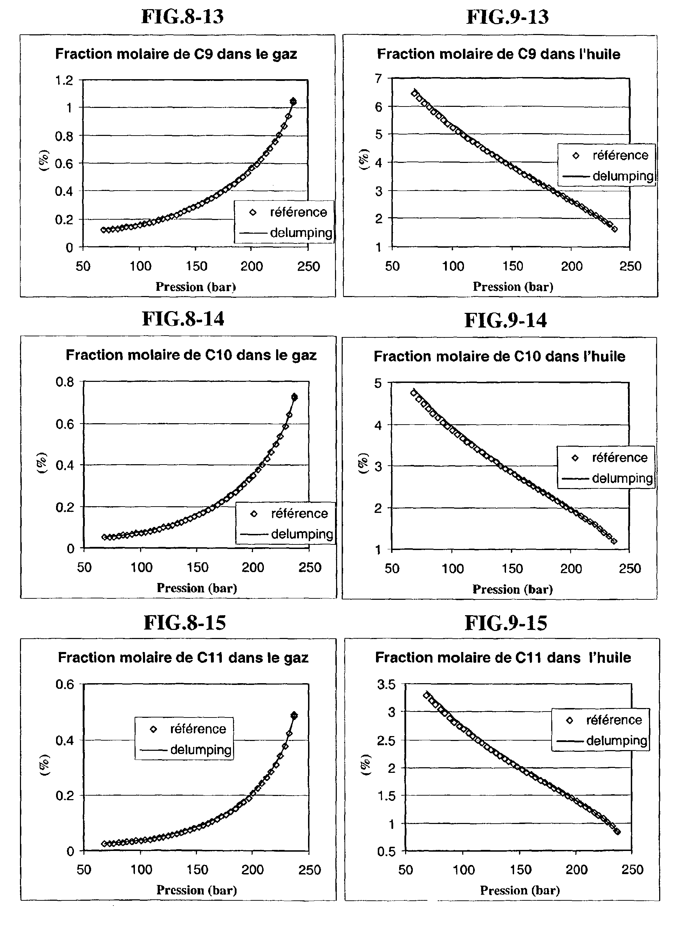

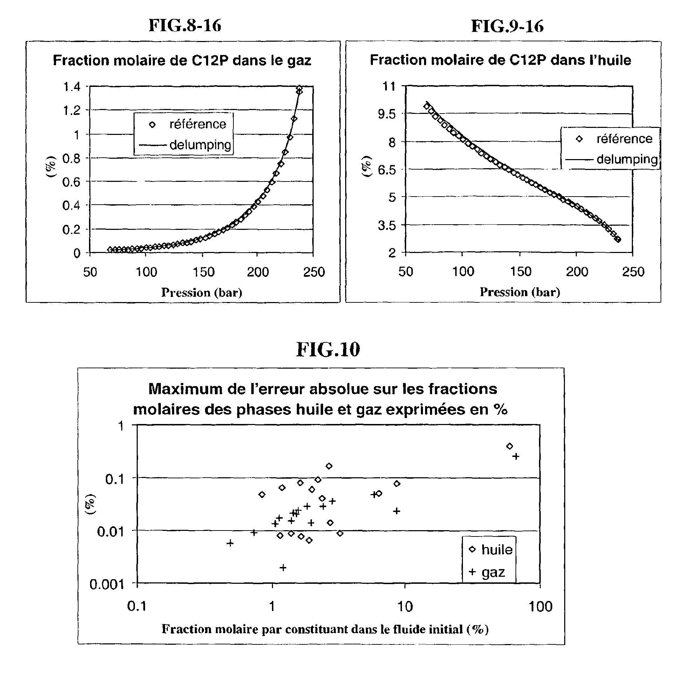

Le papier de W.H. Goldthorpe « Simulation of Gas Injection Processes in Gas-Condensate Reservoirs Using a Binary Pseudo-Component Representation », SPE 19470, présenté à la SPE Asia-Pacific Conference, Sydney, Australie, 13-15 Septembre 1989, illustre les résultats de simulation que l'on peut obtenir avec une modélisation Black-Oil avancée dans un cas d'exploitation d'un réservoir de gaz à condensat par un procédé d'injection de gaz. Le cas pris comme exemple est issu de la publication ci-dessus mentionnée de D.E. Kenyon et G. Alda Behie. Au vu de la disparité des résultats dans cette publication, les résultats de W.H. Goldthorpe avec une représentation Black-Oil, jugée par comparaison avec les résultats d'une simulation effectuée avec une représentation détaillée, apparaissent bien plus cohérents, mais on peut constater dans la figure 8 du papier que la solution n'est pas satisfaisante dans la phase de re vaporisation car la saturation en huile dans une maille particulière (la même que dans le papier de référence) est très différente de celle de la prédiction compositionnelle détaillée, et semble par ailleurs être tronquée de valeurs négatives pendant huit années d'exploitation simulée.

L'état de la technique antérieure est donc que l'on ne dispose pas de méthode de pseudoïsation à la fois simple et robuste:

- permettant de réduire à trois le nombre de pseudo-composants dans des simulations compositionnelles, pour bénéficier de temps de calcul notablement réduits, notamment pour les simulations de cas d'injection de gaz avec effets de re vaporisation, difficilement traitables en Black-Oil,

- garantissant un sens physique aux résultats des simulations compositionnelles et, par voie de conséquence, aux résultats d'opérations associées de delumping.

**LA METHODE SELON L'INVENTION**

La méthode de pseudoïsation selon l'invention permet d'estimer les propriétés ou le comportement des phases hydrocarbure liquide et/ou vapeur, à partir de données relatives à un ensemble de référence constitué de mélanges hydrocarbonés dans une série d'états thermodynamiques découlant de conditions d'exploitation rencontrées, ou prévues comme telles, pour des gisements souterrains d'hydrocarbures. Elle comporte les étapes suivantes :

- on regroupe chacun des dits mélanges hydrocarbonés en au moins trois constituants (V, I, H), aucun de ces constituants ne correspondant à une sélection particulière de composants ou pseudo-composants de base qui serviraient à une description compositionnelle détaillée des fluides, en considérant que les phases gaz issues de la séparation en conditions de surface de chacun des mélanges hydrocarbonés sont des mélanges dont le troisième constituant (H) est exclu, et que les phases huile issues de la séparation en conditions de surface de chacun des mélanges hydrocarbonés sont des mélanges dont le premier constituant (V) est exclu ;

- on détermine par bilan de matière les compositions des produits de séparation comprenant, pour les produits gazeux, au moins le premier et le deuxième constituant (V, I) en proportions variables, et pour les produits liquides, au moins le deuxième et le troisième constituant (I, H) en proportions variables ; et

- on détermine la composition à au moins trois constituants de chaque mélange hydrocarboné de l'ensemble de référence par combinaison des produits de sa séparation au prorata des quantités de chaque produit de séparation.

Suivant un mode de mise en oeuvre, on regroupe chacun des mélanges hydrocarbonés en seulement trois constituants (V, I, H), les phases gaz issues de la dite séparation sont des mélanges en proportions variables du premier constituant (V) et du deuxième constituant (I), on regroupe les phases huile issues de la dite séparation sont des mélanges en proportions variables du deuxième constituant (I) et du troisième constituant (H), et on détermine la composition à trois constituants.

Les conditions de surface sont celles rencontrées ou prévues lors de l'exploitation du gisement mais elles sont redéfinissables selon le contexte de l'étude menée, et peuvent donc être différentes des conditions de surface rencontrées ou prévues lors de l'exploitation du gisement.

Suivant un détail de mise en oeuvre, le bilan de matière est un bilan massique et on attribue à chacun des trois constituants (V, I, H) une masse molaire après une analyse quantitative des masses molaires des produits de séparation de l'ensemble de référence.

Suivant une variante de mise en oeuvre, on définit les données nécessaires aux calculs d'équilibre et à la modélisation des propriétés de phases dans la représentation groupée, en utilisant les compositions des phases dans la représentation groupée et des données connues, ou estimées, a priori portant au moins sur la densité et la viscosité de phases huile et gaz en équilibre appartenant à l'ensemble de référence.

Lorsque les dites données incluent des données compositionnelles détaillées des phases préalablement représentées par une équation d'état « détaillée », on définit les paramètres d'une première (le cas échéant unique) équation d'état de la représentation groupée, utile à la modélisation des propriétés de phases, en utilisant ces données compositionnelles.

Suivant une autre variante préférée de mise en oeuvre, on ajuste les paramètres d'une seconde équation d'état de la représentation groupée, utile aux calculs d'équilibre, pour reproduire les coefficients d'équilibre de la représentation groupée.

Pour réaliser cet ajustement, on utilise par exemple les paramètres par constituant de la représentation groupée dans une équation d'état utile aux calculs des propriétés des phases.

Suivant une autre variante de mise en oeuvre utile en particulier à des calculs ultérieurs d'éclatement ou « delumping », on détermine les coefficients d'équilibre des fluides dans une représentation compositionnelle détaillée, à partir de variables et/ou paramètres intervenant dans le calcul des propriétés de phases, dès lors que les paramètres utiles aux calculs des propriétés de phases dans la représentation groupée ont été estimés de façon à reproduire les paramètres des phases dans l'équation d'état de la description compositionnelle détaillée.

La méthode peut également comporter des étapes d'éclatement ou delumping pour prédire en fonction du temps et dans au moins une zone thermodynamique, une composition détaillée d'un fluide contenu dans un gisement d'hydrocarbures ou produit par au moins un puits, ces étapes étant par exemple les suivantes :

- on représente le réservoir sous la forme d'un réseau de mailles (m) dont chacune constitue un volume élémentaire rempli de fluides sous forme de une ou plusieurs phases, avec au moins une phase non aqueuse ;

- on définit, pour chaque zone ou domaine thermodynamique, les fluides par une représentation détaillée de base, de façon à déterminer la quantité de chaque constituant de base (i) dans chaque phase hydrocarbure dans chaque maille (m) à l'instant défini comme initial pour le calcul de delumping ;

- par zone thermodynamique pour laquelle on adopte une représentation groupée des fluides, on détermine une équation d'état construite, préalablement à la simulation dynamique de réservoir avec la représentation groupée, pour reproduire les paramètres de phases, dans l'équation d'état de la représentation détaillée, des fluides hydrocarbonés au cours de chemins thermodynamiques tenus pour représentatifs de ceux que vont suivre les fluides hydrocarbonés pendant la simulation maillée ;

- on réalise à un pas de temps t une simulation compositionnelle avec un nombre limité de constituants où les propriétés des phases sont calculées par une équation d'état, ladite simulation permettant de calculer au moins dans chaque maille (m) et à des pas de temps consécutifs, une pression pour une phase hydrocarbure, la température lorsque celle-ci varie, les débits des phases entre mailles, entre mailles perforées et puits, et les valeurs de paramètres et/ou propriétés de phase intervenant dans l'expression formelle des coefficients d'équilibre de la représentation détaillée, et on mémorise ces diverses quantités ;

- on estime au pas de temps suivant (t+1) la fraction molaire de chaque constituant i dans la composition détaillée globale du fluide hydrocarboné dans la maille (m) par bilan matière sur la maille (m) ;

- on détermine en utilisant les quantités mémorisées, au même pas de temps (t+1) et dans chaque maille (m), les coefficients d'équilibre de chaque constituant (i) dans la représentation détaillée ;

- on détermine, au même pas de temps (t+1), la fraction vaporisée dans chaque maille (m) ; et

- on estime la composition détaillée de chaque phase hydrocarbonée, au même pas de temps (t+1) et dans chaque maille (m) en particulier sous la forme des nombres de moles de chaque constituant i dans la représentation détaillée dans chaque phase au même pas de temps (t+1).

En d'autres termes, on peut aussi définir l'essentiel de la méthode de pseudoïsation comme suit :

- Ayant à disposition des données, au moins de densités et viscosités, concernant des phases huile et gaz en équilibre « in situ » pour un certain nombre d'états thermodynamiques 'e' dans des conditions de pression et température $P^e$ et $T^e$,

- connaissant directement ou par l'intermédiaire de calculs préliminaires, les masses molaires des phases 'huile' en provenant de la séparation de l'huile in situ, de la séparation du gaz in situ, notées respectivement $(MM_{OO}^e)$ et $(MM_{GO}^e)$, les masses molaires des phases gaz en surface provenant de la séparation de l'huile in situ, de la séparation du gaz in situ, notées respectivement $(MM_{OG}^e)$ et $(MM_{GG}^e)$, et les fractions molaires vapeur (ou fraction molaire de phase gaz), $\theta_{OO}^e$ pour la séparation de l'huile in situ, $\theta_{GO}^e$ pour la séparation du gaz in situ,

- on détermine les compositions des phases « en surface », et par voie de conséquence les coefficients d'équilibre, dans la représentation groupée utilisant les constituants (V, I, H) avec l'hypothèse que les phases gaz en surface peuvent contenir les constituants (V) et (I), à l'exclusion du constituant (H), et que les phases huile en surface peuvent contenir les constituants (I) et (H), à l'exclusion du constituant (V).

- on détermine les compositions des phases « in situ » dans la représentation groupée utilisant les constituants (V, I, H) par recombinaison des compositions des phases en surface en utilisant les fractions molaires vapeur $\theta_{OO}^e$, $\theta_{GO}^e$, ces déterminations fournissant les coefficients d'équilibre des constituants (V, I, H) dans les conditions de réservoir « in-situ »,

- on utilise les données connues a priori et les compositions des phases dans la représentation groupée pour définir les entrées nécessaires à la modélisation des propriétés de phases et aux calculs d'équilibre dans la représentation groupée et, plus particulièrement lorsque les données a priori incluent des données compositionnelles détaillées des phases préalablement représentées par une équation d'état, on utilise ces informations additionnelles pour définir les paramètres d'une équation d'état de la représentation groupée utile en premier lieu à la modélisation des propriétés de phases, le cas échéant pour définir les paramètres d'une seconde équation d'état de la représentation groupée utile aux calculs d'équilibre.

Les constituants V, I, H s'apparentent respectivement à un constituant volatile, un constituant intermédiaire et un constituant lourd, sans qu'aucun d'entre eux ne corresponde à une sélection particulière de composants ou pseudo-composants de base qui serviraient à une description compositionnelle détaillée des fluides.

L'idée essentielle mise en oeuvre pour le dégroupage ou delumping des simulations compositionnelles est de calculer les coefficients d'équilibre des fluides dans la représentation compositionnelle détaillée à partir de variables et/ou paramètres intervenant dans le calcul des propriétés de phases dès lors que les paramètres utiles aux calculs des propriétés de phases dans la représentation groupée ont été estimés de façon à reproduire les paramètres des phases dans l'équation d'état de la description compositionnelle détaillée

## PRESENTATION DES FIGURES

D'autres caractéristiques et avantages de la méthode selon l'invention, apparaîtront à la lecture de la description ci-après d'un exemple non limitatif de réalisation, en se référant aux dessins annexés présentés où :

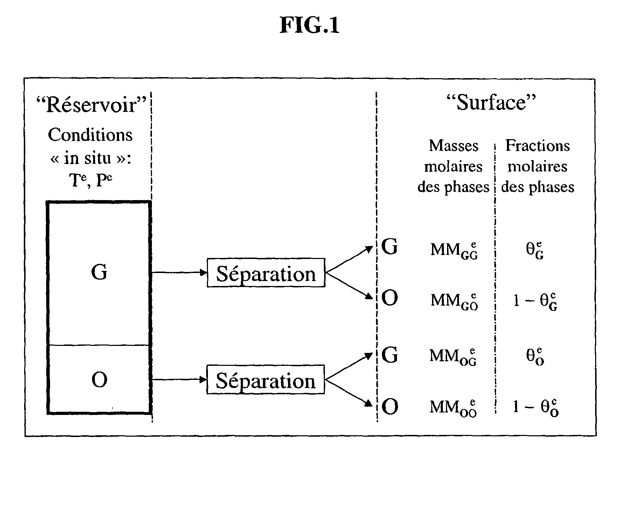

- la figure 1 schématise la séparation distincte d'une phase huile et d'une phase gaz en équilibre dans le réservoir ;

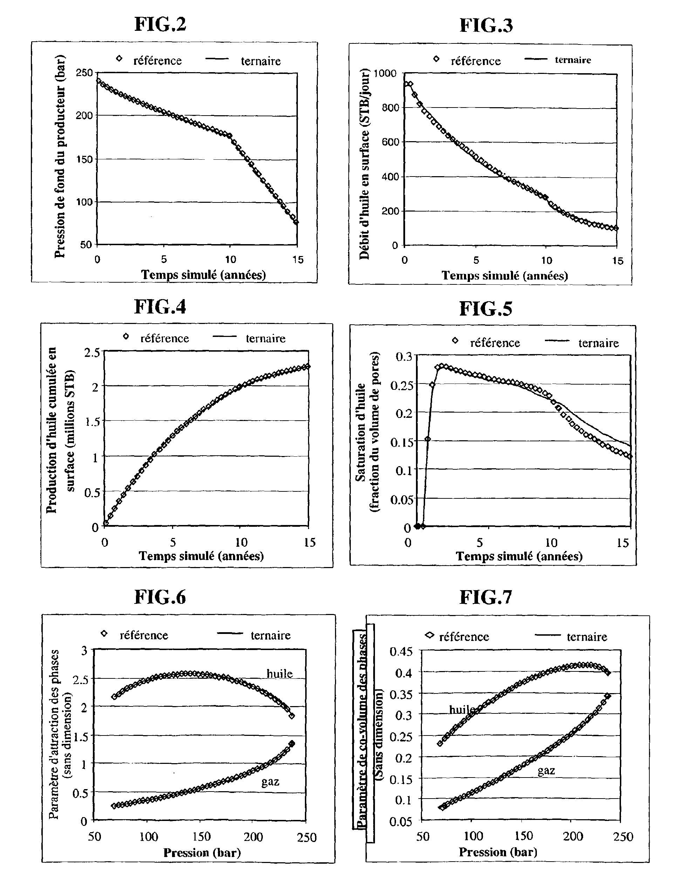

- les figures 2 à 5 comparent les résultats d'une simulation compositionnelle avec une représentation à trois pseudo-composants (résultats désignés par 'ternaire'), obtenue par application de la méthode selon l'invention, à ceux d'une simulation compositionnelle détaillée à seize constituants de base (résultats désignés par 'référence ;

- les figures 6 et 7 montrent, pour les représentations détaillée et ternaire, respectivement les variations du terme d'attraction et du co-volume (exprimés sous forme adimensionnelle) des phases huile et gaz en fonction de la pression au cours de la simulation thermodynamique d'une opération de dépressurisation à volume constant à la température de réservoir ;

- les figures 8.1 à 8.16 d'une part, et les figures 9.1 à 9.16 d'autre part, montrent, par constituant, respectivement la composition détaillée du gaz et de l'huile, au cours de la même opération de dépressurisation, telles qu'obtenues à partir de la représentation de référence, et à partir de l'éclatement de la représentation à trois pseudo-composants ;

- la figure 10 récapitule les résultats illustrés aux figures 8.1 à 8.16 et 9.1 à 9.16, en montrant l'erreur absolue maximale obtenue sur la teneur en chaque constituant durant la dépressurisation de 237.4 à 69 bars en fonction de la teneur du constituant dans la phase à la pression de rosée du fluide initial ;

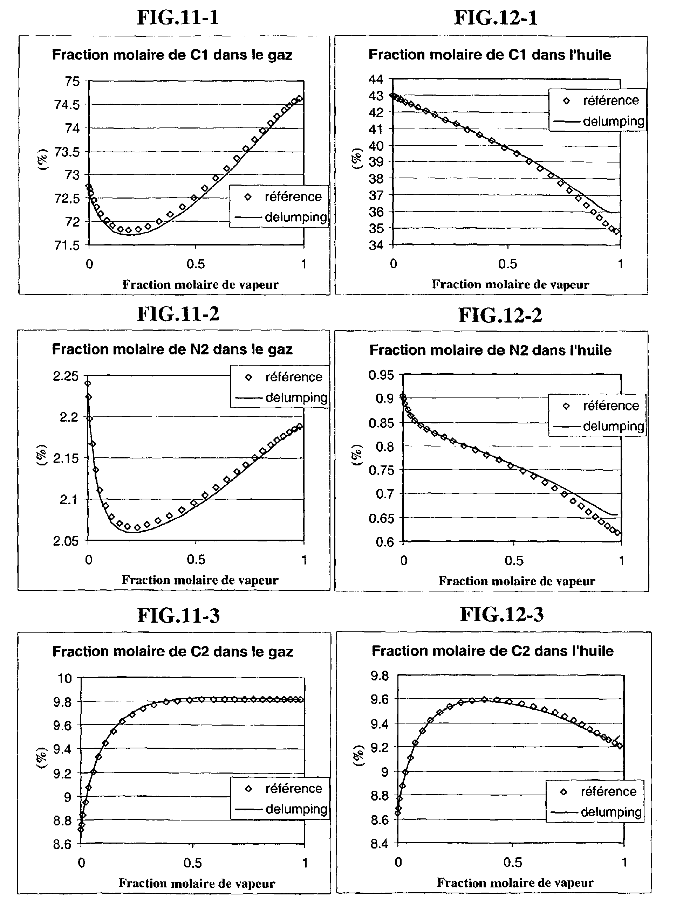

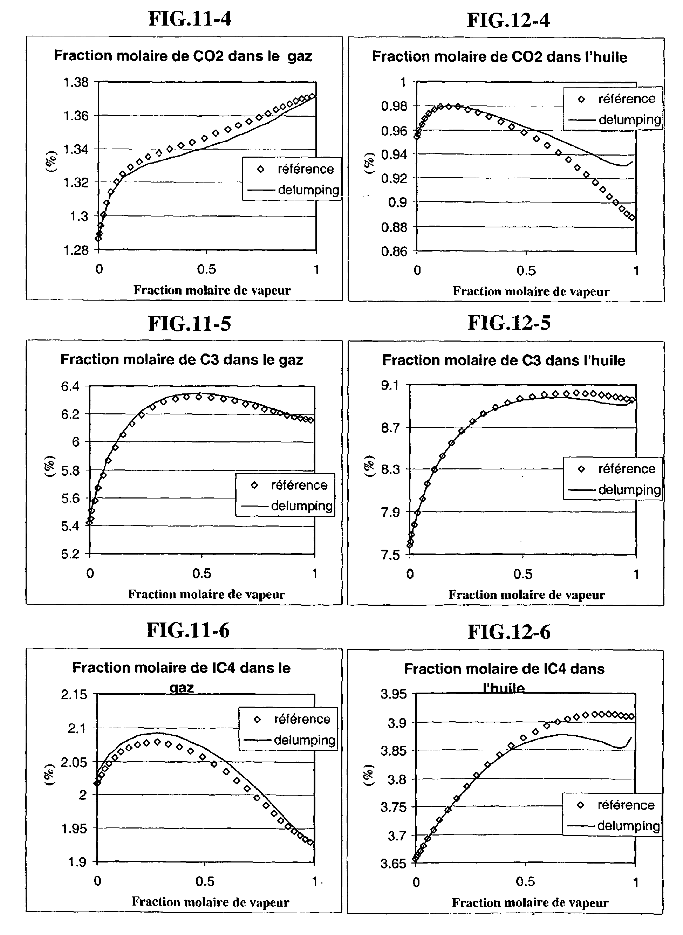

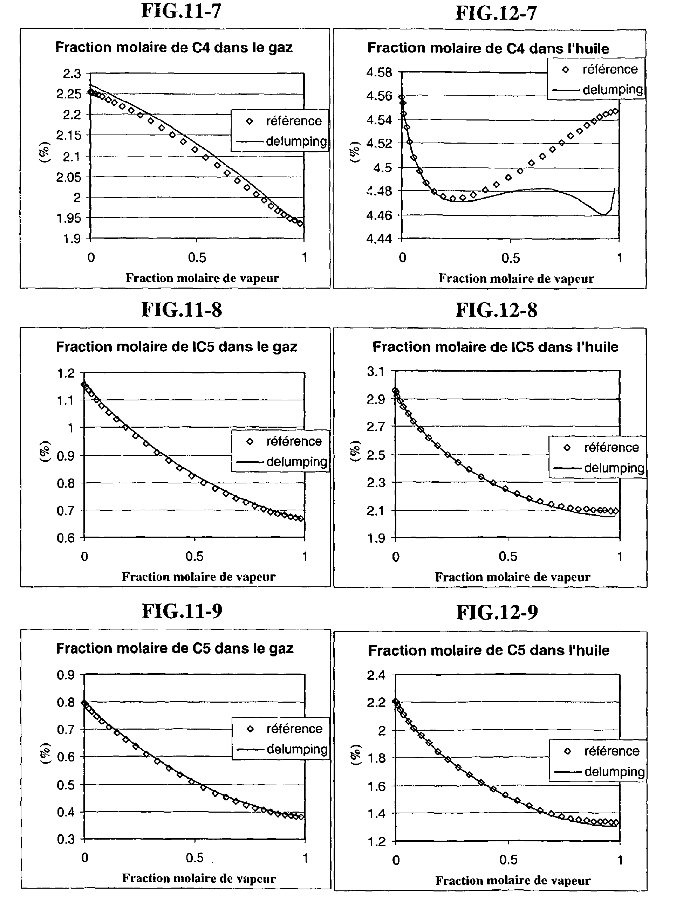

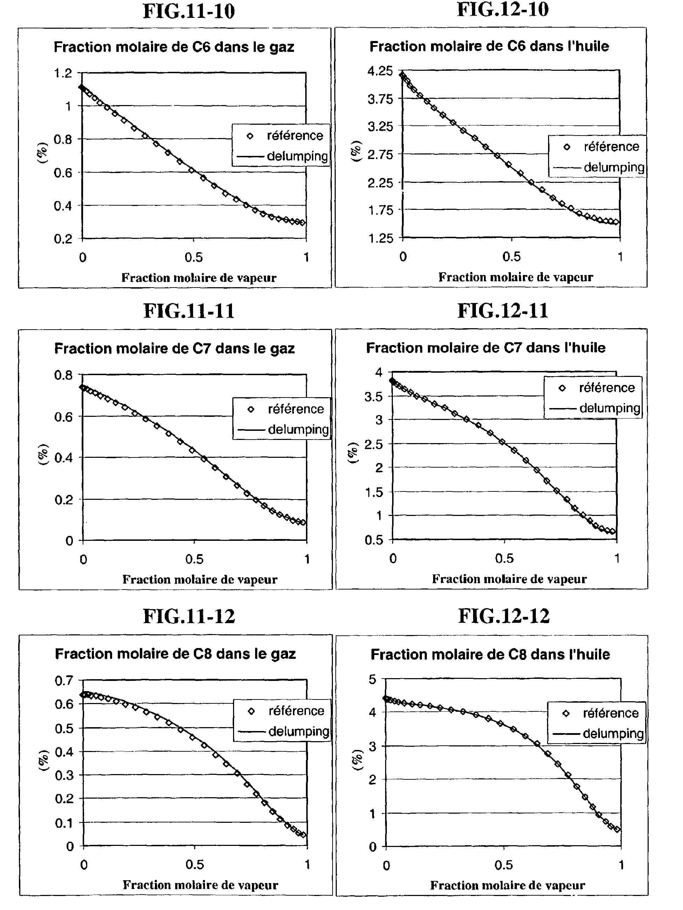

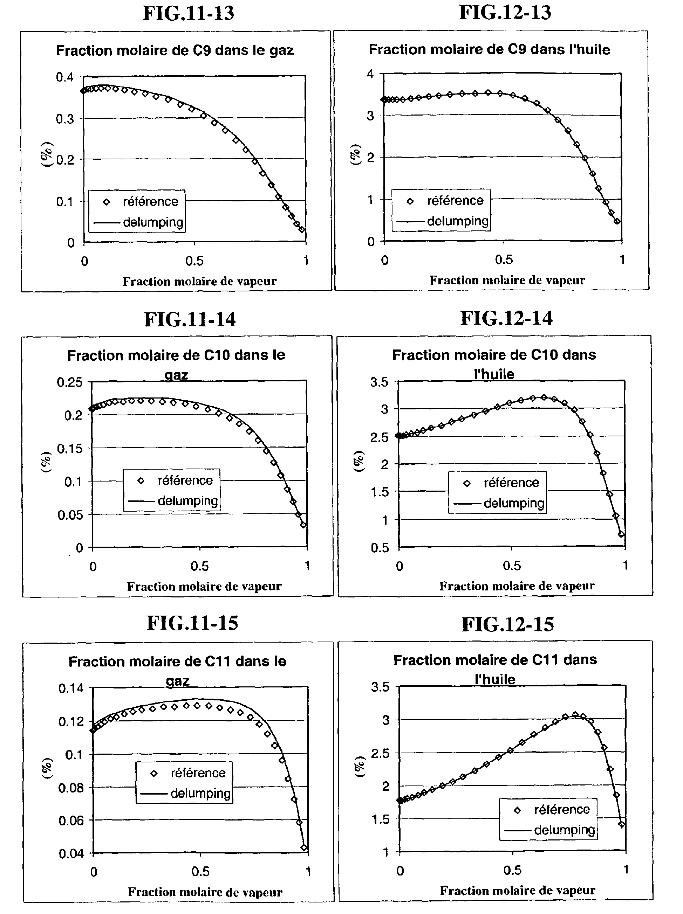

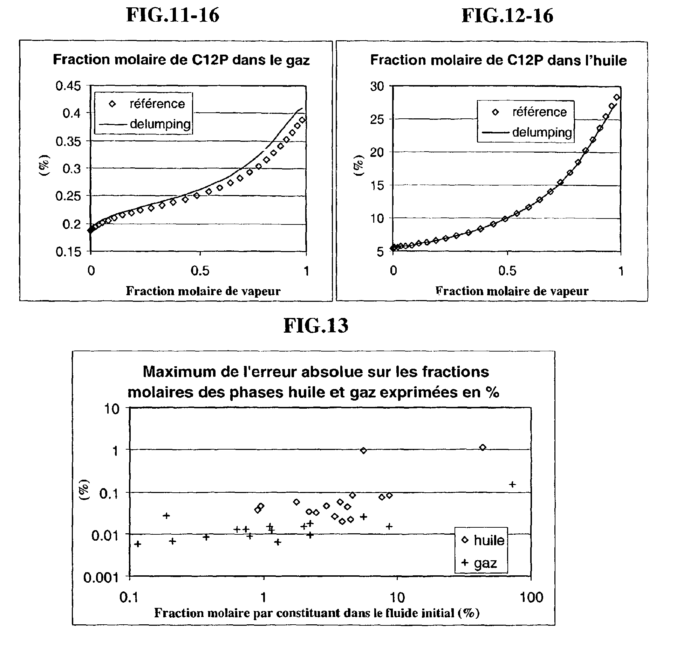

- les figures 11.1 à 11.16 d'une part, et les figures 12.1 à 12.16 d'autre part, montrent, par constituant, respectivement la composition détaillée du gaz et de l'huile, au cours de la simulation thermodynamique d'une opération de vaporisation d'un condensat par injection d'un gaz sec à la pression constante de 169 bars, telles qu'obtenues à partir de la représentation de référence, et à partir de l'éclatement de la représentation à trois pseudo-composants ; et

- la figures 13 récapitule les résultats illustrés aux figures 11.1 à 11.16 et 12.1 à 12.16, en montrant l'erreur absolue maximale obtenue sur la teneur en chaque constituant pour l'opération de vaporisation en fonction de la teneur du constituant dans la phase au démarrage de l'opération de vaporisation.

## DESCRIPTION DETAILLEE

*Préambule*

La modélisation du comportement de mélanges fluides nécessite un mode de définition des constituants utilisés pour décrire la composition des fluides et comporte :

- des calculs d'équilibre thermodynamique, pour savoir si, dans des conditions données de pression et de température, l'état d'équilibre d'un mélange, caractérisé par une composition globale, est monophasique, ou si le mélange se sépare en différentes phases, le calcul d'équilibre permettant alors de connaître les proportions de chaque phase, et la composition de chaque phase. Un calcul d'équilibre sur un mélange, dont la composition globale est décrite par les fractions molaires $z_i$ de chaque constituant (i), conduit à la détermination de la fraction vapeur $\theta$ et à la détermination de la composition de chaque phase. La fraction vapeur $\theta$ est déterminée en général en résolvant l'équation de Rachford-Rice connue des spécialistes qui est appliquée par exemple dans le document suivant :

- Rachford, H.H.Jr et Rice, J.D.; "Procedure for Use of Electronic Digital Computers in Calculating Flash Vaporization Hydrocarbon Equilibrium", J. Pet. Technol., 1952, **14**, 19,

$$(2) \qquad \sum_i \frac{z_i\,(K_i - 1)}{1 + (K_i - 1)\,\theta} = 0$$

les compositions des phases 'huile' et 'gaz', décrites respectivement par les fractions molaires $x_i$ et $y_i$, étant déterminées par:

$$(3) \qquad x_i = \frac{z_i}{1 + (K_i - 1)\,\theta} \qquad ; \qquad y_i = \frac{K_i\,z_i}{1 + (K_i - 1)\,\theta}$$

Une phase hydrocarbure est dite sous-saturée à une pression et une température T donnée lorsqu'elle ne se trouve pas en équilibre avec une autre phase hydrocarbure. Selon une convention souvent admise par les gens de l'art, une phase hydrocarbure sous-saturée est dite 'huile' (ou 'condensat') lorsque sa température critique est inférieure à la température T, et est dite 'gaz' lorsque sa température critique est supérieure à la température T. Lorsque sa température critique est égale à la température T, la phase peut être qualifiée, en principe indifféremment, de 'huile' ou 'gaz', et est donc considérée comme rattachée à l'une ou l'autre des qualités 'huile' ou 'gaz'.

- des calculs de propriétés des phases telles que masse volumique, viscosité,

- le cas échéant, des calculs, dits de delumping, permettant la restitution d'une information compositionnelle détaillée lorsque les fluides ont été modélisés de façon non détaillée.

Les différentes étapes de la méthode de modélisation proposée sont présentées ci-après, la présentation étant structurée en cinq sous-chapitres:

- mode de définition des constituants,
- propriétés des phases,
- équilibres thermodynamiques,
- delumping,
- remarques concernant la généralisation de l'approche.

*Mode de définition des constituants*

a) On considère un ensemble « E », dit de référence, de fluides non aqueux, dits mélanges hydrocarbure même s'ils peuvent contenir certains composants autres que des hydrocarbures tel que l'azote, le dioxyde de carbone, l'anhydride sulfureux, susceptibles de correspondre :

- aux phases fluides hydrocarbure sous-saturées trouvées, supposées ou prédites dans une ou plusieurs zones thermodynamiques du réservoir considéré ;

- aux phases fluides hydrocarbure en équilibre trouvées, supposées ou prédites dans une ou plusieurs zones thermodynamiques du réservoir considéré ;

- aux phases fluides hydrocarbure correspondant aux fluides hydrocarbure injectés (ou supposés l'être) dans le réservoir. Dans les cas pratiques les plus fréquents, les fluides d'injection sont des gaz contenant principalement des hydrocarbures légers et intermédiaires.

b) Lorsque l'on dispose des données nécessaires, on définit une représentation détaillée à $N_{rb}$ composants et/ou pseudo-composants de base pour caractériser les mélanges fluides. Cette situation étant très courante, on place la suite de l'exposé dans une telle situation car cela permet d'introduire progressivement les conventions d'usage utiles à la description de la méthode.
Le nombre $N_{rb}$ étant typiquement supérieur à 10, chaque constituant est décrit par un certain nombre de paramètres, dont sa masse molaire $MM_i$, l'indice i étant le numéro attribué au constituant ( i=1,..., $N_{rb}$). Les fractions molaires $x_i$ et $y_i$ décrivent respectivement la composition des phases hydrocarbure liquide ('huile') et vapeur ('gaz') dans la représentation de base.
Un élément 'e' de l'ensemble « E » est alors décrit par : une pression $P^e$, une température $T^e$, et:

- (2 x $N_{rb}$) fractions molaires $\left( x_1^e \ .. \ x_{N_{rb}}^e \right)$ et $\left( y_1^e \ .. \ y_{N_{rb}}^e \right)$ lorsque deux phases hydrocarbure en équilibre coexistent,

- ou ($N_{rb}$) fractions molaires $\left( x_1^e \ .. \ x_{N_{rb}}^e \right)$ ou $\left( y_1^e \ .. \ y_{N_{rb}}^e \right)$ lorsque une seule phase hydrocarbure (huile ou gaz) sous-saturée est présente.

Dans ce qui suit, on écrit les équations pour un élément 'e' où deux phases liquide et vapeur coexistent. Pour un élément 'e', où une seule phase sous-saturée est présente, il suffit de sélectionner le jeu d'équations relatives à la seule phase représentée.

c) On considère que l'on dispose, pour chaque phase de l'ensemble « E » de référence, de données de fluides susceptibles de correspondre aux compositions des phases fluides hydrocarbure trouvées, supposées ou prédites correspondant aux mélanges fluides produits par une chaîne de séparation, la chaîne de séparation pouvant être réduite à une seule détente ou comporter un étage ou plusieurs étages intermédiaires de séparation. Une chaîne de séparation est ici, comme il est d'usage dans l'ingénierie de gisement, considérée comme définie par les valeurs de pressions et températures des étages de séparation. Il est pratique de considérer la chaîne de séparation existante ou prévue par laquelle le mélange fluide est ou est supposé être produit. On peut être amené, dans des cas pratiques, à considérer différentes chaînes de séparation, par exemple une chaîne de séparation différente par zone thermodynamique, ou un changement de chaîne de séparation dans une zone thermodynamique donnée. Dans ce qui suit, on simplifie d'abord la présentation en ne considérant qu'une chaîne de séparation identique pour tous les éléments de l'ensemble « E », puis on considère le cas de plusieurs chaînes de séparation.

Les produits de séparation d'une phase huile de composition $(x_1^e .. x_{Nrb}^e)$ sont en général une phase liquide (phase huile) et une phase vapeur (phase gaz), dont les masses molaires sont supposées connues et désignées respectivement par $MM_{OO}^e$ et $MM_{OG}^e$, dans les proportions molaires supposées connues de $\theta_O^e$ de phase vapeur et $(1 - \theta_O^e)$ de phase liquide. $\theta_O^e$ peut prendre toutes les valeurs réelles entre 0.0 et 1.0, bornes incluses. L'indice O (en caractère gras) rappelle qu'il s'agit des produits de séparation de la phase huile. Les indices O et G (en caractère non gras) donnent la qualité huile ou gaz du produit de séparation.

Les produits de séparation d'une phase gaz de composition $(y_1^e .. y_{Nrb}^e)$ sont en général une phase liquide (phase huile) et une phase vapeur (phase gaz), dont les masses molaires sont supposées connues et désignées respectivement par $MM_{GO}^e$ et $MM_{GG}^e$, dans les proportions molaires supposées connues de $\theta_G^e$ de phase vapeur et $(1 - \theta_G^e)$ de phase liquide. $\theta_G^e$ peut prendre toutes les valeurs réelles entre 0.0 et 1.0, bornes incluses. L'indice G (en caractère gras) rappelle qu'il s'agit des produits de séparation de la phase gaz.

La Fig. 1 schématise la séparation distincte d'une phase huile et d'une phase gaz en équilibre dans le réservoir dans la situation générale où la séparation (simple ou complexe) d'un mélange initialement monophasique, liquide (huile) ou gaz, produit deux phases, l'une gazeuse, l'autre liquide.

Les relations suivantes traduisent la conservation de la masse lors des opérations de séparation respectivement de l'huile et/ou du gaz de l'élément 'e':

$$(4) \quad \begin{cases} MM_O^e = (1 - \theta_O^e)\, MM_{OO}^e + \theta_O^e\, MM_{OG}^e \\[2em] MM_G^e = (1 - \theta_G^e)\, MM_{GO}^e + \theta_G^e\, MM_{GG}^e \end{cases}$$

où $MM_O^e$ et $MM_G^e$ sont les masses molaires des phases huile et gaz d'un élément 'e'.

d) La méthode de pseudoïsation proposée consiste à décrire chaque phase hydrocarbonée de l'ensemble « **E** » en au moins trois constituants (V, I, H).

On définit les pseudo-composants en considérant une hypothèse désignée ci-après par « **M** », selon laquelle les phases gaz issues de la séparation en conditions de surface de chacun des mélanges hydrocarbonés sont des mélanges dont le troisième constituant (H) est exclu, et que les phases huile issues de la séparation en conditions de surface de chacun des mélanges hydrocarbonés sont des mélanges dont le premier constituant (V) est exclu.

Les constituants (V), (I), (H) s'apparentent respectivement à un constituant volatile, intermédiaire et lourd, sans qu'aucun d'entre eux ne corresponde à une sélection particulière de composants ou pseudo-composants de base qui serviraient à une description compositionnelle détaillée des fluides. La modélisation associée est désignée ci-après en abrégé par BRO, pour 'BROWN OIL', pour évocation de la couleur de l'huile qui passe du noir au marron avec un contenu croissant en constituants intermédiaires.

En un premier temps, pour faciliter l'exposé, on considère selon une interprétation restrictive de l'hypothèse maîtresse

«*M*» que les mélanges produits par la chaîne de séparation sont:

- un mélange en proportions variables des pseudo constituants volatile (V) et intermédiaire (I) pour un produit gazeux (la masse molaire du constituant V étant inférieure à celle du constituant I) ;

- et un mélange en proportions variables des pseudo constituants intermédiaire (I) et lourd (H) pour un produit liquide (la masse molaire du constituant I étant inférieure à celle du constituant H).

La restriction sur l'hypothèse « *M* » sera levée dans le paragraphe tt).
Les relations suivantes s'appliquent donc:

$$(5) \quad \begin{cases} MM_{OO}^{e} = x_{OI}^{e} MM_{I} + x_{OH}^{e} MM_{H} & ; & x_{OI}^{e} + x_{OH}^{e} = 1 \\[2mm] MM_{OG}^{e} = y_{OV}^{e} MM_{V} + y_{OI}^{e} MM_{I} & ; & y_{OV}^{e} + y_{OI}^{e} = 1 \\[2mm] MM_{GO}^{e} = x_{GI}^{e} MM_{I} + x_{GH}^{e} MM_{H} & ; & x_{GI}^{e} + x_{GH}^{e} = 1 \\[2mm] MM_{GG}^{e} = y_{GV}^{e} MM_{V} + y_{GI}^{e} MM_{I} & ; & y_{GV}^{e} + y_{GI}^{e} = 1 \end{cases}$$

Pour conserver un sens physique à la représentation ternaire, il est nécessaire que:

- les masses molaires $MM_V$, $MM_I$, $MM_H$ soient définies positives ;

- les fractions molaires $x_{OI}^{e}$ , $x_{OH}^{e}$ , $y_{OV}^{e}$ , $y_{OI}^{e}$ , $x_{GI}^{e}$ , $x_{GH}^{e}$ , $y_{GV}^{e}$ , $y_{GI}^{e}$ soient comprises entre 0.0 et 1.0, bornes incluses.

Il en découle que le choix des masses molaires $MM_V$, $MM_I$, $MM_H$ doit respecter les inégalités suivantes:

- 
$$MM_V \le Min \, \{MM_{GG}^{e}, MM_{OG}^{e}\}\Big|_{(e \in E)}$$

- 
$$Max \, \{MM_{GG}^{e}, MM_{OG}^{e}\}\Big|_{(e \in E)} \le MM_I \le Min \, \{MM_{GO}^{e}, MM_{OO}^{e}\}\Big|_{(e \in E)}$$

- 
$$MM_H \ge Max \, \{MM_{GO}^{e}, MM_{OO}^{e}\}\Big|_{(e \in E)}$$

Dans les équations précédentes, les accolades désignent un ensemble de valeurs sur lesquelles on recherche une valeur minimum (Min) ou maximum (Max).

Les inégalités étant non strictes, l'absence de l'un des constituants (V,I) dans un produit de séparation gazeux donné est possible mais ne constitue pas une règle générale et, de même, l'absence de l'un des constituants (I,H) dans un produit de séparation liquide donné est possible mais ne constitue pas une règle générale.

e) Les valeurs des masses molaires des constituants (V), (I), (H) doivent être choisies en respectant les conditions ci-dessus et être toutes différentes les unes des autres. Dans l'exemple fourni à titre d'illustration, on a posé, par analogie avec une démarche Black-Oil, et sans que ce soit un exemple limitatif de réalisation:

- 

$$MM_V \equiv Min \left.\{MM_{GG}^e, MM_{OG}^e\}\right|_{(e \in E)} \quad ; \quad MM_H \equiv Max \left.\{MM_{GO}^e, MM_{OO}^e\}\right|_{(e \in E)}$$

L'exemple d'illustration, largement utilisé dans la suite de la description sous la désignation de cas 'SPE3', correspond au premier cas test de la publication déjà citée de D.E. Kenyon et G. Alda Behie d'exploitation d'un gisement de gaz à condensat par cyclage de gaz.

f) Une fois les masses molaires des constituants (V), (I) et (H) définies, la composition des fluides issus de la chaîne de séparation considérée en c) se déduisent, par les hypothèses du paragraphe d) et les équations (**5**), par:

$$(6) \begin{cases} x_{OV}^e = 0 \quad ; \quad x_{OI}^e = \dfrac{MM_H - MM_{OO}^e}{MM_H - MM_I} \quad ; \quad x_{OH}^e = \dfrac{MM_{OO}^e - MM_I}{MM_H - MM_I} \\[3mm] y_{OV}^e = \dfrac{MM_I - MM_{OG}^e}{MM_I - MM_V} \quad ; \quad y_{OI}^e = \dfrac{MM_{OG}^e - MM_V}{MM_I - MM_V} \quad ; \quad y_{OH}^e = 0 \\[3mm] x_{GV}^e = 0 \quad ; \quad x_{GI}^e = \dfrac{MM_H - MM_{GO}^e}{MM_H - MM_I} \quad ; \quad x_{GH}^e = \dfrac{MM_{GO}^e - MM_I}{MM_H - MM_I} \end{cases}$$

$$y_{GV}^e = \dfrac{MM_I - MM_{GG}^e}{MM_I - MM_V} \quad ; \quad y_{GI}^e = \dfrac{MM_{GG}^e - MM_V}{MM_I - MM_V} \quad ; \quad y_{GH}^e = 0$$

g) Pour obtenir la composition des phases de l'élément 'e' dans la représentation à trois constituants, on utilise les relations suivantes (dans lesquelles une notation en caractères gras est adoptée pour désigner les fractions molaires des fluides in situ) qui traduisent la conservation molaire lors des opérations de séparation respectivement de l'huile et/ou du gaz de l'élément 'e':

$$(7) \begin{cases} \mathbf{x}_K^e = (1 - \theta_O^e) x_{OK}^e + \theta_O^e y_{OK}^e \\[3mm] \mathbf{y}_K^e = (1 - \theta_G^e) x_{GK}^e + \theta_G^e y_{GK}^e \end{cases} \qquad K = V, I, H$$

En introduisant les relations (**6**) dans le jeu d'équations (**7**), on obtient dans le réservoir pour une phase huile de l'élément 'e' la composition suivante:

$$(8) \quad \begin{cases} x_V^e = \theta_O^e \dfrac{MM_I - MM_{OG}^e}{MM_I - MM_V} \\[3mm] x_I^e = (1 - \theta_O^e) \dfrac{MM_H - MM_{OO}^e}{MM_H - MM_I} + \theta_O^e \dfrac{MM_{OG}^e - MM_V}{MM_I - MM_V} \\[3mm] x_H^e = (1 - \theta_O^e) \dfrac{MM_{OO}^e - MM_I}{MM_H - MM_I} \end{cases}$$

et pour une phase gaz:

$$(9) \quad \begin{cases} y_V^e = \theta_G^e \dfrac{MM_I - MM_{GG}^e}{MM_I - MM_V} \\[3mm] y_I^e = (1 - \theta_G^e) \dfrac{MM_H - MM_{GO}^e}{MM_H - MM_I} + \theta_G^e \dfrac{MM_{GG}^e - MM_V}{MM_I - MM_V} \\[3mm] y_H^e = (1 - \theta_G^e) \dfrac{MM_{GO}^e - MM_I}{MM_H - MM_I} \end{cases}$$

h) A l'issue des étapes a) à g), on dispose donc, pour une utilisation ultérieure, des données minimales pour définir les constituants (V), (I), (H) à savoir leurs masses molaires.

On note que, pour l'ensemble des éléments 'e' correspondant à des états d'équilibre entre phases hydrocarbure, on dispose, d'après les jeux d'équations (8) et (9), des coefficients d'équilibre de chacun des constituants (V), (I), (H):

$$(10) \quad \begin{cases} K_V^e = \dfrac{y_V^e}{x_V^e} = \dfrac{\theta_G^e}{\theta_O^e} \dfrac{MM_I - MM_{GG}^e}{MM_I - MM_{OG}^e} \\[4mm] K_I^e = \dfrac{y_I^e}{x_I^e} = \dfrac{(1 - \theta_G^e) \dfrac{MM_H - MM_{GO}^e}{MM_H - MM_I} + \theta_G^e \dfrac{MM_{GG}^e - MM_V}{MM_I - MM_V}}{(1 - \theta_O^e) \dfrac{MM_H - MM_{OO}^e}{MM_H - MM_I} + \theta_O^e \dfrac{MM_{OG}^e - MM_V}{MM_I - MM_V}} \\[6mm] K_H^e = \dfrac{y_H^e}{x_H^e} = \dfrac{1 - \theta_G^e}{1 - \theta_O^e} \dfrac{MM_{GO}^e - MM_I}{MM_{OO}^e - MM_I} \end{cases}$$

On note également que le jeu d'équations (6) définit les coefficients d'équilibre pour les conditions de sortie, en pression et température, de la chaîne de séparation considérée dans le paragraphe c):

$$(11) \quad \begin{cases} K_{OV}^e = \dfrac{y_{OV}^e}{x_{OV}^e} = \infty \quad ; \quad K_{OI}^e = \dfrac{y_{OI}^e}{x_{OI}^e} = \dfrac{MM_{OG}^e - MM_V}{MM_H - MM_{OO}^e}\dfrac{MM_H - MM_I}{MM_I - MM_V} \quad ; \quad K_{OH}^e = \dfrac{y_{OH}^e}{x_{OH}^e} = 0 \\[3em] K_{GV}^e = \dfrac{y_{GV}^e}{x_{GV}^e} = \infty \quad ; \quad K_{GI}^e = \dfrac{y_{GI}^e}{x_{GI}^e} = \dfrac{MM_{GG}^e - MM_V}{MM_H - MM_{GO}^e}\dfrac{MM_H - MM_I}{MM_I - MM_V} \quad ; \quad K_{GH}^e = \dfrac{y_{GH}^e}{x_{GH}^e} = 0 \end{cases}$$

i) Le jeu d'équations (**7**), écrit pour respectivement les constituants (V) et (H), donne les relations suivantes:

$$(12) \quad \begin{cases} x_V^e = \theta_O^e \, y_{OV}^e \quad ; \quad x_H^e = (1 - \theta_O^e) \, x_{OH}^e \\[1.5em] y_V^e = \theta_G^e \, y_{GV}^e \quad ; \quad y_H^e = (1 - \theta_G^e) \, x_{GH}^e \end{cases}$$

dont il découle:

$$(13) \quad \begin{cases} y_{OV}^e = \dfrac{x_V^e}{\theta_O^e} \quad ; \quad x_{OH}^e = \dfrac{x_H^e}{1 - \theta_O^e} \\[2em] y_{GV}^e = \dfrac{y_V^e}{\theta_G^e} \quad ; \quad x_{GH}^e = \dfrac{y_H^e}{1 - \theta_G^e} \end{cases}$$

puis:

$$(14) \quad \begin{cases} y_{OI}^e = 1 - \dfrac{x_V^e}{\theta_O^e} \quad ; \quad x_{OI}^e = 1 - \dfrac{x_H^e}{1 - \theta_O^e} \quad ; \quad K_{OI}^e = \dfrac{1 - \dfrac{x_V^e}{\theta_O^e}}{1 - \dfrac{x_H^e}{1 - \theta_O^e}} \\[3em] y_{GI}^e = 1 - \dfrac{y_V^e}{\theta_G^e} \quad ; \quad x_{GI}^e = 1 - \dfrac{y_H^e}{1 - \theta_G^e} \quad ; \quad K_{GI}^e = \dfrac{1 - \dfrac{y_V^e}{\theta_G^e}}{1 - \dfrac{y_H^e}{1 - \theta_G^e}} \end{cases}$$

Le jeu d'équations (**14**) montre que, dès lors que les masses molaires des constituants (V), (I), (H) sont fixées, et que les compositions des phases d'un élément 'e' dans la représentation ternaire ont été établies, les coefficients d'équilibre du constituant (I) peuvent être obtenus à partir de la seule connaissance des fractions molaires des constituants (V) et (H) dans les phases huile et gaz de l'élément 'e' et des fractions vapeur $\theta_O^e$ et $\theta_G^e$ en sortie de chaîne de séparation pour chacune des phases. Une première implication est que, en cas de chaînes de séparation multiples, il est possible de générer les données de coefficients d'équilibre du constituant (I) relative à une autre chaîne de séparation (ci-après désignée par CS_B) que celle utilisée pour obtenir les compositions huile et gaz de l'élément 'e' (ci-après désignée par CS_A). Ceci est schématisé dans le tableau suivant :

| Mélange détaillé | Opération | Données utilisées | Mélange ternaire | Coefficients d'équilibre CS_B |
|---|---|---|---|---|
| $(x_1^e .. x_{N_{rb}}^e)_{\searrow}^{\nearrow}$ | CS_A  -------> | $\{MM_{OO}^e, MM_{OG}^e, \theta_O^e\}_{CS\_A}$  --------------------> | $\{x_V^e, x_I^e, x_H^e\}_{CS\_A}$ | $K_{OI}^e = \dfrac{1 - \dfrac{x_{V\,CS\_A}^e}{\theta_{O\,CS\_B}^e}}{1 - \dfrac{x_{H\,CS\_A}^e}{1 - \theta_{O\,CS\_B}^e}}$ |
| | CS_B  -------> | $\{\theta_O^e\}_{CS\_B}$  ------------------> | -------> | |
| $(y_1^e .. y_{N_{rb}}^e)_{\searrow}^{\nearrow}$ | CS_A  -------> | $\{MM_{GO}^e, MM_{GG}^e, \theta_G^e\}_{CS\_A}$  --------------------> | $\{y_V^e, y_I^e, y_H^e\}_{CS\_A}$ | $K_{GI}^e = \dfrac{1 - \dfrac{y_{V\,CS\_A}^e}{\theta_{G\,CS\_B}^e}}{1 - \dfrac{y_{H\,CS\_A}^e}{1 - \theta_{G\,CS\_B}^e}}$ |
| | CS_B  -------> | $\{\theta_G^e\}_{CS\_B}$  ------------------> | -------> | |

Les conditions nécessaires à une telle procédure sont le respect des inégalités suivantes:

- $y_{OI\,CS\_B}^e \geq 0$ et $x_{OI\,CS\_B}^e \geq 0$ et d'où $x_{V\,CS\_A}^e \leq \theta_{O\,CS\_B}^e \leq 1 - x_{H\,CS\_A}^e$

- $y_{GI\,CS\_B}^e \geq 0$ et $x_{GI\,CS\_B}^e \geq 0$ et d'où $y_{V\,CS\_A}^e \leq \theta_{G\,CS\_B}^e \leq 1 - y_{H\,CS\_A}^e$

Il convient de noter que les coefficients d'équilibre du constituant (I) ainsi établis ne sont pas formellement équivalents à ceux que l'on obtiendrait en appliquant les jeux d'équations (**6**) à (**11**) à la chaîne de séparation CS_B, moyennant la connaissance des masses molaires $\{MM_{GG}^e, MM_{OO}^e, MM_{OG}^e, MM_{GO}^e\}_{CS\_B}$, ces masses molaires devant satisfaire, vis à vis des masses molaires des constituants (V), (I), (H), les inégalités décrites dans le paragraphe d). On peut, si besoin est, réviser le choix des masses molaires des constituants (V), (I), (H), en tenant compte des masses molaires des phases issues de la séparation CS_B dans l'écriture des inégalités du paragraphe d).

j) A titre d'exemple, la démarche non limitative de réalisation suivie pour obtenir la représentation BRO pour le cas 'SPE3' présenté en illustration a consisté en différentes étapes:

- générer avec un simulateur thermodynamique tel que par exemple le simulateur dit 'PVT PACKAGE') un ensemble d'états 'e_d' par une opération de dépressurisation à volume constant à la température du réservoir (93.3 °C), à partir de la pression de saturation du fluide (237.4 bar) jusqu'à 69 bar, en utilisant une équation d'état de Peng-Robinson, d'emploi très largement répandu dans le contexte pétrolier, pour le fluide détaillé à 16 constituants dont la composition initiale et les paramètres sont donnés dans le Tableau 1 ci-après :

| Tableau 1 Cas SPE3-Description de référence du fluid de réservoir | | | | | | | | | | | | |
|---|---|---|---|---|---|---|---|---|---|---|---|---|
| Nom | Fraction molaire | MM | $T_c$ ˚F | $P_c$ psi | $V_c$ ft$^3$/lbm | $\omega$ | $\delta_{KK'}$ (·) | N2 | ci | CO2 | C2 | C3 |
| N2 | .0194 | 28.016 | -232.33 | 492.84 | 1.4433 | 0.0350 | N2 | 0.000 | # | # | # | # |
| C1 | .6599 | 16.043 | -115.76 | 672.98 | 1.5858 | 0.0130 | C1 | 0.120 | 0.000 | # | # | # |
| CO2 | .0121 | 44.010 | 88.07 | 1069.7 | 1.5057 | 0.2250 | CO2 | 0.000 | 0.15000 | 0.000 | # | # |
| C2 | .0869 | 30.070 | 90.34 | 709.67 | 2.3707 | 0.1050 | C2 | 0.120 | 0.00000 | 0.150 | 0.000 | # |
| C3 | .0591 | 44.097 | 206.28 | 617.28 | 3.2037 | 0.1520 | C3 | 0.120 | 0.00000 | 0.150 | 0.000 | 0.000 |
| IC4 | .0239 | 58.124 | 274.96 | 528.95 | 4.2129 | 0.1918 | IC4 | 0.120 | 0.00000 | 0.150 | 0.000 | 0.000 |
| C4 | .0278 | 58.124 | 305.64 | 550.56 | 4.1007 | 0.2010 | C4 | 0.120 | 0.00000 | 0.150 | 0.000 | 0.000 |
| IC5 | .0157 | 72.151 | 370.11 | 483.41 | 4.9337 | 0.2060 | IC5 | 0.120 | 0.00000 | 0.150 | 0.000 | 0.000 |
| C5 | .0112 | 72.151 | 385.92 | 489.36 | 4.9817 | 0.2520 | C5 | 0.120 | 0.00000 | 0.150 | 0.000 | 0.000 |
| C6 | .0181 | 84.000 | 452.25 | 468.47 | 5.9268 | 0.2809 | C6 | 0.120 | 0.03000 | 0.150 | 0.010 | 0.010 |
| C7 | .0144 | 100.205 | 512.64 | 396.68 | 6.9200 | 0.3520 | C7 | 0.120 | 0.03000 | 0.150 | 0.010 | 0.010 |
| C8 | .0150 | 114.232 | 564.64 | 362.02 | 7.8811 | 0.3992 | C8 | 0.120 | 0.03200 | 0.150 | 0.010 | 0.010 |
| C9 | .0105 | 128.259 | 613.31 | 331.99 | 8.7621 | 0.4439 | C9 | 0.120 | 0.03400 | 0.150 | 0.010 | 0.010 |
| C10 | .0073 | 142.286 | 655.07 | 304.15 | 9.6591 | 0.4869 | C10 | 0.120 | 0.03600 | 0.150 | 0.010 | 0.010 |
| C 11 | .0049 | 147.000 | 699.91 | 349.54 | 10.172 | 0.4770 | C 11 | 0.120 | 0.04300 | 0.150 | 0.010 | 0.010 |
| C12P | .0138 | 210.000 | 797.52 | 324.79 | 14.000 | 0.5266 | C12P | 0.120 | 0.04408 | 0.150 | 0.010 | 0.010 |

Dans ce tableau, les paramètres d'interactions binaires forment une matrice symétrique de trace nulle ; les cases où figurent un symbole # sont remplies par symétrie. Les colonnes manquantes doivent être remplies d'une part par la propriété de symétrie, d'autre part par des paramètres d'interactions binaires nuls.

- A divers paliers de dépressurisation répartis entre la pression de saturation du fluide initial et 69 bar, on a effectué une opération de vaporisation à pression constante (la pression du palier considéré) de la phase huile de l'état 'e$_d$' considéré par un gaz dont la composition est tenue pour représentative du gaz d'injection en cours de simulation (dans le cas SPE3, la composition du gaz injecté varie en cours de simulation puisque c'est le gaz produit qui est réinjecté), chaque opération de vaporisation comportant plusieurs paliers de quantités de gaz injecté pour aller jusqu'à la vaporisation complète de la phase liquide. A chaque palier de quantité de gaz injecté où les phases sont saturées correspond un état 'e$_{dv}$'.

- Toujours avec le même simulateur, les opérations de séparation des phases huile et gaz de chacun des états 'e$_d$' et 'e$_{dv}$' formant l'ensemble « E » ont été simulées, la chaîne de séparation CS_A utilisée comportant un premier étage à 26.7 °C et 56.2 bar, un second étage de détente de la phase liquide issue du premier étage de séparation à 26.7 °C et 4.5 bar, une dernière détente de la phase liquide issue du deuxième étage de séparation à 15.6 °C et 1.01325 bar, les phases gazeuses issues des différentes séparations étant mélangées. Comme le cas SPE3 présente une modification, au cours de la simulation, des conditions de pression du premier étage de séparation (la pression passant de 56.2 bar à 21.7 bar), les opérations de séparation des phases huile et gaz de chacun des états 'e' ont également été simulées avec cette autre chaîne de séparation CS_B.

Il convient de noter que l'enchaînement des diverses opérations de simulation ci-dessus est aisément réalisable avec la plupart des logiciels de simulations thermodynamiques disponibles pour l'industrie.

Les conditions nécessaires décrites dans le paragraphe d) trouvées à partir des masses molaires des produits de séparation des phases huile et gaz de chacun des états de l'ensemble « E » par les chaînes CS_A et CS_B sont en grammes/mole:

- $MM_V \leq 21.592$ ; $27.865 \leq MM_I \leq 72.334$ ; $MM_H \geq 162.06$

Les masses molaires attribuées aux trois constituants dans la représentation BRO sont données dans le Tableau 2. Les compositions à trois constituants des phases de l'ensemble « E » par les calculs décrits dans les paragraphes f) et g) ont été obtenues en considérant les produits de séparation de la chaîne CS_A. La composition du fluide initial dans la représentation BRO est donnée dans le Tableau 2 ci-après :

| Tableau 2: Cas SPE3 - Description BRO du fluide de réservoir | | |
|---|---|---|
| Nom du constituant | MM | Fraction molaire |
| V | 21.592 | .856714 |
| I | 62.334 | .092358 |
| H | 162.06 | .050928 |

*Propriétés des phases*

La règle de Gibbs, qui donne le nombre de degrés de liberté d'un système thermodynamique à partir du nombre de constituants et du nombre de phases, appliquée à un mélange à trois constituants, donne trois degrés de liberté lorsque deux phases hydrocarbure sont à l'équilibre : les propriétés de phases dépendent de la pression, la température et d'une variable compositionnelle.

Pour chaque phase fluide pour laquelle on connaît, pour des conditions de pression et température données, les valeurs de propriétés de phase utiles pour la modélisation des écoulements (facteur de compressibilité, viscosité, ..), on peut rentrer ces propriétés de phase sous forme de tables fonctions de la pression, de la température, et d'un index de la composition dans la représentation à trois constituants. Ces données peuvent être utilisées ensuite comme données d'entrées dans un simulateur, le simulateur disposant en général de méthodes internes d'interpolation ou d'extrapolation pour estimer les propriétés de phases en des points intermédiaires.

k) Alternativement à l'usage de tables, on peut rechercher, par exemple par régression, les paramètres de corrélations ou d'équations d'état permettant de calculer, par zone thermodynamique, les propriétés des phases en fonction de la composition et des conditions de pression et de température, ces paramètres étant ensuite introduits dans le modèle de simulation en lieu et place des tables précédemment citées.

Une situation fréquente est de disposer d'une équation d'état fiable pour le fluide de réservoir décrit de façon détaillée dans la représentation de base.

Ainsi, lorsqu'une équation d'état (et une corrélation associée pour le calcul des viscosités) définie pour la représentation détaillée à $N_{rb}$ constituants, permet de modéliser le comportement thermodynamique des fluides au cours des étapes de pressions et températures d'un chemin thermodynamique suivi dans une ou plusieurs zones thermodynamiques, on peut utiliser cette équation d'état pour générer l'ensemble de référence de compositions de fluides et les propriétés déjà citées des phases correspondantes, en y adjoignant les paramètres des phases dans l'équation d'état, tels que le co-volume, le terme d'attraction, la pression critique, qui peuvent être considérés comme des propriétés spécifiques des phases. Pour chacune des compositions de fluides obtenues, cette même équation d'état, ou une autre, peut être utilisée pour générer les compositions et propriétés des mélanges fluides produits par une chaîne de séparation, et permettre ainsi l'application de la méthode de pseudoïsation décrite dans les paragraphes a) à i).

Lorsque les paramètres des pseudo-composants dans une équation d'état, et dans une corrélation pour le calcul de viscosité, sont ajustés, par exemple par régression, pour reproduire les propriétés spécifiques des phases telles que le co-volume, le terme d'attraction, la température critique, .. obtenues avec la représentation détaillée, on obtient une équation d'état et une corrélation utilisable pour les calculs de propriétés de phase telles que le facteur de compressibilité, la viscosité, mais cette première équation d'état, désignée ci-après en abrégé par EOS_PRO, n'est pas forcément valide pour les calculs d'équilibre notamment si le respect d'une condition d'égalité des fugacités à l'équilibre n'a pas été pris en compte.

Une démarche non limitative de réalisation pour obtenir les paramètres de l'équation d'état EOS_PRO de la représentation BRO est celle suivie pour le cas 'SPE3' présenté en illustration. L'équation d'état utilisée, celle de Peng-Robinson, est rappelée ci-après pour aider à la compréhension de la démarche, mais la démarche est aisément transposable à d'autres équations d'état.

l) L'équation de Peng-Robinson s'écrit pour la phase P :

$$(15) \quad P = \frac{R\,T}{V_P - b_P} - \frac{a(T)_P}{(V_P + b_P)^2 - 2\,b_P^2}$$

où R est la constante des gaz parfaits, T la température, P la pression, V le volume molaire de la phase P, b son co-volume et a(T) son terme d'attraction, ces dernières quantités étant exprimées par :

$$(16) \quad b_P = \sum_i c_i\,b_i$$

$$(17) \quad a(T)_P = \sum_j c_j \left[ \sum_i c_i\,a_{ij}(T) \right] \text{avec } a_{ij}(T) = \sqrt{a_i(T)}\,\sqrt{a_j(T)}\,(1 - \delta_{ij})$$

où $C_i$ désigne la fraction molaire du constituant i dans la phase P, fraction molaire dont les dépendances physiques sont omises pour alléger la présentation, $b_i$ et $a_i(T)$ respectivement le co-volume et le terme d'attraction du constituant i, $\delta_{ij}$ le paramètre d'interaction binaire entre les constituants i et j, les $\delta_{ij}$ formant une matrice symétrique de trace nulle. Sous forme adimensionnelle, l'équation de Peng-Robinson s'exprime comme:

$$(18) \quad Z_P^3 - (1 - B_P)\,Z_P^2 + (A_P - 3\,B_P^2 - 2\,B_P)\,Z_P - (A_P\,B_P - B_P^2 - B_P^3) = 0$$

où Z est le facteur de compressibilité de la phase P, et B et A sont les formes adimensionnelles des co-volume et terme d'attraction de la phase P (les dépendances des variables avec la pression et température étant omises pour alléger l'écriture des équations):

$$(19) \quad B_P = b_P \frac{P}{R\,T}$$

$$(20) \quad A_P = a_P \frac{P}{R^2\,T^2}$$

m) Le facteur de compressibilité de la phase P est uniquement fonction des deux paramètres de phase adimensionnels A et B. Il s'ensuit qu'estimer les paramètres par constituant dans la représentation BRO (à savoir $b_V$, $b_I$, $b_H$, $a_V(T)$, $a_I(T)$, $a_H(T)$, $\delta_{VI}$, $\delta_{VH}$, $\delta_{IH}$) permettant de reproduire les valeurs de $a(T)$ et $b$, obtenues pour chaque phase aux différents paliers des simulations PVT avec la représentation de base (détaillée à 16 constituants), permet de reproduire les valeurs de référence des facteurs de compressibilité des phases. La procédure de régression utilisée pour obtenir les différents paramètres de la représentation BRO est la minimisation de fonctions objectif de la forme:

$$(21) \quad O(\vec{p}) = \sum_{e}^{e\in E} \varpi_G^e \, [\,\varphi_G^e(\vec{p}) - \, o_G^e\,]^2 + \sum_{e}^{e\in E} \varpi_O^e \, [\,\varphi_O^e(\vec{p}) - \, o_O^e\,]^2$$

où la quantité observable o par phase et par état e, ici obtenue avec la représentation détaillée à 16 constituants, est modélisée par une fonctionnelle $\varphi$ paramétrée par les composantes $p_\pi$ du vecteur $\vec{p}$, et où les quantités $\tilde{\omega}$ sont des poids attribués aux différentes observables.

Le minimum de $O(\vec{p})$ est obtenu par la résolution du système d'équations, de même dimension que le vecteur $\vec{p}$, formé par la nullité des dérivées partielles de la fonction $O(\vec{p})$ par rapport aux différents paramètres $p_\pi$ :

$$(22) \quad \partial_{p_\pi} O(\vec{p}) = 0$$

soit:

$$(23) \quad \sum_{e}^{e\in E} \varpi_G^e \, [\,\varphi_G^e(\vec{p}) - \, o_G^e\,]\, \partial_{p_\pi}\varphi_G^e(\vec{p}) + \sum_{e}^{e\in E} \varpi_O^e \, [\,\varphi_O^e(\vec{p}) - \, o_O^e\,]\, \partial_{p_\pi}\varphi_O^e(\vec{p}) = 0$$

n) Pour la recherche des valeurs des paramètres $b_K$ (K=V,I,H) de la représentation BRO, la fonction objectif utilisée dans le cas SPE3 est donc:

$$(24) \quad O(b_V, b_I, b_H) = \sum_e^{e \in E} \varpi_G^e \left[ \sum_K y_K^e b_K - b_G^e \right]^2 + \sum_e^{e \in E} \varpi_O^e \left[ \sum_K x_K^e b_K - b_O^e \right]^2$$

Le système à résoudre est simplement un système linéaire de trois équations à trois inconnues:

$$(25) \quad \begin{cases} m_{VV}\, b_V + m_{VI}\, b_I + m_{VH}\, b_H = r_V \\ m_{IV}\, b_V + m_{II}\, b_I + m_{IH}\, b_H = r_I \\ m_{HV}\, b_V + m_{HI}\, b_I + m_{HH}\, b_H = r_H \end{cases}$$

les termes $m_{KK'}$ et $r_K$ des seconds membres des équations étant donnés par :

$$m_{KK'} = \sum_e^{e \in E} (\varpi_G^e\, y_K^e\, y_{K'}^e + \varpi_O^e\, x_K^e\, x_{K'}^e) \; ; \qquad r_K = \sum_e^{e \in E} (\varpi_O^e\, x_K^e\, b_O^e + \varpi_G^e\, y_K^e\, b_G^e)$$

o) Pour rechercher les paramètres $a_V(T)$, $a_I(T)$, $a_H(T)$, $\delta_{VI}$, $\delta_{VH}$, $\delta_{IH}$ par simplement des résolutions de systèmes linéaires de trois équations à trois inconnues, l'équation (**17**) peut être réécrite comme la somme de deux termes, le second terme comprenant les interactions binaires :

$$(26) \quad a(T)_P = a_1(T)_P{}^2 + a_2(T)_P$$

avec :

$$(27) \quad a_1(T)_P = \sum_i c_i \sqrt{a_i(T)}$$

et

$$a_2(T)_P = - \sum_j \sum_i c_j\, c_i\, \alpha_{ij} \text{ où } \quad \alpha_{ij}(T) = \sqrt{a_i(T)}\, \sqrt{a_j(T)}\, \delta_{ij}$$

Dans le terme $a_2(T)_P$, les paramètres $\alpha_{ij}(T)$ forment un ensemble de trois paramètres indépendants $\alpha_{VI}(T)$, $\alpha_{VH}(T)$, $\alpha_{IH}(T)$. En utilisant les termes $a_1(T)$, obtenus lors des simulations PVT avec la représentation détaillée à 16 constituants, comme quantités observables o par phase et par état e, les inconnues étant les quantités $\sqrt{a_V(T)}$, $\sqrt{a_I(T)}$, $\sqrt{a_H(T)}$, le problème à résoudre est bien analogue à celui décrit pour l'obtention des paramètres de co-volumes des constituants de la représentation BRO. De même, les paramètres $\alpha_{ij}(T)$ sont obtenus en résolvant le système de trois équations à trois inconnues formé à partir des observables $a_2(T)$.

p) Les paramètres d'interactions binaires peuvent être ensuite obtenus par:

**(28)**

$$\delta_{VI} = \frac{\alpha_{VI}(T)}{\sqrt{a_V(T)}\,\sqrt{a_I(T)}} \quad ; \qquad \delta_{VH} = \frac{\alpha_{VH}(T)}{\sqrt{a_V(T)}\,\sqrt{a_H(T)}} \qquad ; \qquad \delta_{IH} = \frac{\alpha_{IH}(T)}{\sqrt{a_I(T)}\,\sqrt{a_H(T)}}$$

q) Certains simulateurs acceptent, comme paramètres des constituants pour l'équation d'état, l'introduction directe des co-volumes $b_i$ des constituants et de la matrice des paramètres $a_{ij}(T)$ intervenant dans le calcul du terme d'attraction (cf. l'équation (**17**)), cette matrice devant être définie le cas échéant pour différentes températures.

Au cas où le cas traité nécessiterait une dépendance en température (température variable dans le réservoir), la démarche précédente pourrait être adaptée, par exemple en générant l'ensemble « E » par des opérations PVT à diverses températures de réservoir, en considérant des états e à diverses températures pour obtenir les co-volumes par constituant (ceux-ci devant être les mêmes pour les différentes températures), en considérant les états e obtenus à une température donnée pour générer la matrice des paramètres $a_{ij}(T^e)$ correspondante et en répétant la démarche pour autant de températures $T^e$ que nécessaire.

r) Lorsque le simulateur n'accepte pas l'introduction directe du co-volume par constituant $b_i$ et des matrices de paramètres $a_{ij}(T^e)$:

- le co-volume par constituant est en général obtenu à partir de l'introduction des paramètres $Tc_i$, $Pc_i$, optionnellement $\Omega b_i$ (par défaut tous égaux à 0.077796) par :

$$(29) \quad b_i = R\,\Omega b_i\,\frac{Tc_i}{Pc_i}$$

- les quantités $a_i(T)$ sont en général obtenues par:

$$(30) \quad \sqrt{a_i(T)} = \sqrt{a0_i}\left[\,1 + \zeta_i\left(1 - \sqrt{\frac{T}{Tc_i}}\right)\right] \qquad \text{avec} \qquad \zeta_i = \sum_{n=0}^{dp} C_n\,\omega_i^{\,n}$$

La quantité $a0_i$ est en général obtenue à partir de l'introduction des paramètres $Tc_i$, $Pc_i$, optionnellement $\Omega a_i$ (par défaut tous égaux à 0.457235) par:

$$(31) \quad a0_i = R^2\,\Omega a_i\,\frac{Tc_i^{\,2}}{Pc_i}$$

Dans le polynôme $\zeta_i$ de degré dp, les paramètres $\omega_i$ sont les facteurs acentriques des constituants en général considérés comme constants, le degré du polynôme et les constantes $C_n$ pouvant varier d'un simulateur à l'autre.

s) Lorsque les pressions, températures et volumes critiques par constituant sont requis, on peut utiliser comme observables les pressions, températures et volumes critiques des phases de l'ensemble « E », définies par des lois de mélanges identiques à celles utilisées pour les co-volumes :

$$(32) \quad Pc_P = \sum_i c_i \, Pc_i \qquad ; \qquad Tc_P = \sum_i c_i \, Tc_i \qquad ; \qquad Vc_P = \sum_i c_i \, Vc_i$$

L'obtention respectivement des paramètres $Pc_V$, $Pc_I$, $Pc_H$, $Tc_V$, $Tc_I$, $Tc_H$, et $Vc_V$, $Vc_I$, $Vc_H$ peut donc s'effectuer par résolution successive de trois systèmes de trois équations à trois inconnues totalement analogues à ceux générés pour le calcul des co-volumes par constituant, développé précédemment.

t) Les paramètres $\Omega b_K$ (K=V,I,H) sont alors obtenus, à partir de l'équation (29) et des paramètres $b_K$, $Pc_K$ et $Tc_K$ tels que déterminés dans les paragraphes n) et s), par:

$$(33) \quad \Omega b_K = \frac{Pc_K \, b_K}{R \, Tc_K}$$

u) Dans l'équation (30), dans le cas le plus fréquent où la température de réservoir est considérée comme constante, on peut s'affranchir de la dépendance en température des quantités $\sqrt{a_V(T)}, \ \sqrt{a_I(T)}, \ \sqrt{a_H(T)},$ en adoptant une valeur nulle pour les facteurs acentriques des constituants V, I, H, d'où:

$$(34) \quad \sqrt{a_K(T)} \equiv \sqrt{a0_K} \ , \ \forall K, \ K=V,I,H$$

et on obtient alors les paramètres $\Omega a_K$ (K=V,I,H), à partir des paramètres $\sqrt{a_K(T)},$ $Pc_K$ et $Tc_K$ tels que déterminés dans les paragraphes o) et s), par:

$$(35) \quad \Omega a_K = \frac{Pc_K \, a_K(T)}{R^2 \, Tc_K^2}$$

Une autre possibilité, utilisée dans le cas SPE3, est l'attribution aux paramètres $\Omega a_K$ (K=V,I,H) de la valeur par défaut. Dans ce cas, les paramètres $\sqrt{a0_K}$ sont déterminés avec les $Pc_K$ et $Tc_K$ tels que déterminés dans le paragraphe s), et les paramètres $\zeta_K$ sont ensuite obtenus par réarrangement de l'équation (30):

$$(36) \quad \zeta_K = \frac{\dfrac{\sqrt{a_K(T)}}{\sqrt{a0_K}} - 1}{1 - \sqrt{\dfrac{T}{Tc_K}}}$$

puis, les facteurs acentriques $\omega_K$ ont été recherchés comme solutions des trois équations polynomiales (K=V,I,H):

$$(37) \quad \sum_{n=0}^{dp} C_n \, \omega_K^{\ n} - \zeta_K = 0$$

v) Lorsque pour le cas considéré, la température du réservoir est variable, on peut réécrire l'équation (**30**) comme :

$$(38) \quad \sqrt{a_K(T^e)} = \beta_K + \gamma_K \, \eta_K(T^e)$$

avec les quantités : $\eta_K(T^e) = 1 - \sqrt{\dfrac{T^e}{Tc_K}}$ , calculées en utilisant les températures critiques des constituants telles que déterminées dans le paragraphe s).

On peut ensuite rechercher les quantités $\beta_K$ et $\Upsilon_K$ ($\beta_K = \sqrt{a0_K}$ et $\gamma_K = \sqrt{a0_K} \, \zeta_K$) comme des paramètres indépendants par régression linéaire des couples de valeurs $\eta_K(T^e)$, $\sqrt{a_K(T^e)}$, ces dernières valeurs étant telles que déterminées dans les paragraphes o) et q).

Les paramètres $\Omega a_K$ et $\zeta_K$ sont alors obtenus par:

$$(39) \quad \Omega a_K = \frac{Pc_K \, \beta_K^{\ 2}}{R^2 \, Tc_K^{\ 2}} \qquad \text{et} \qquad \zeta_K = \frac{\gamma_K}{\beta_K}$$

Les facteurs acentriques recherchés sont solutions des trois équations polynomiales (K=V,I,H):

$$(40) \quad \sum_{n=0}^{dp} C_n \, \omega_K^{\ n} - \frac{\gamma_K}{\beta_K} = 0$$

w) Des facteurs de correction de volume sont souvent utilisés pour ajuster les densités des phases. Lorsque des facteurs de correction de volume par constituant sont requis, on peut utiliser comme observables les corrections de volume des phases de l'ensemble « E », définies par des lois de mélanges identiques à celles utilisées pour les co-volumes:

$$(41) \quad Cv_P = \sum_i c_i \, Cv_i$$

L'obtention des paramètres $Cv_V$, $Cv_I$, $Cv_H$, peut s'effectuer alors par résolution d'un système de trois équations à trois inconnues totalement analogue à celui généré pour le calcul des co-volumes par constituant développé dans le paragraphe n).

x) Dans le cas SPE3, les calculs de viscosité des phases hydrocarbure ont été effectués avec une corrélation largement utilisée dans le domaine de l'ingénierie pétrolière, celle de Lohrenz, John, Bray, B.G. et Clark, C.R.: « Calculating Viscosities of Reservoir Fluids from Their Compositions » , Journal of Petroleum Technology, 1964, pp. 1171-1176. Les paramètres de phases utilisés explicitement dans cette corrélation sont les pressions, températures, volumes critiques des phases. La détermination des paramètres critiques (pression, température, volume) des constituants V, I, H, telle qu'exposée dans le paragraphe s), a permis une modélisation appropriée des viscosités des phases avec la représentation BRO. La démarche est aisément transposable à d'autres corrélations.

y) La ligne directrice suivie dans l'ensemble des paragraphes n) à w) est de rechercher les paramètres d'équation d'état EOS_PRO utiles aux calculs, dans les conditions de réservoir, de densités des phases (via les facteurs de compressibilité) et de viscosités par des résolutions simples (non itératives) de systèmes d'équations linéaires.

Son efficacité pour reproduire les co-volumes et termes d'attraction des phases est illustrée, pour les fluides du cas SPE3, par les Fig. 6 et 7 pour une opération de dépressurisation à volume constant à la température de réservoir (93.33 ˚C) à partir de la pression de rosée bars du fluide de réservoir initial (237.4 bars) jusqu'à 69 bars. Sur chacune des figures, sont illustrées les quantités calculées à partir de l'équation d'état de la représentation détaillée de référence et les quantités calculées à partir de l'équation d'état EOS_PRO de la modélisation BRO, ces quantités étant pratiquement confondues.

Cette même ligne a été utilisée avec le même succès pour traiter d'autres cas non présentés ici. On a pu constater qu'une telle ligne est largement adaptable à des contextes de température variable. Par ailleurs, les traitements nécessaires ne représentent aucune difficulté particulière de codage et ont été aisément automatisables.

Les paramètres de l'EOS_PRO utilisés pour le cas SPE3 en illustration sont donnés dans le tableau 3 ci-dessous :

| Tableau 3: Cas SPE3 - Paramètres de l'EOS_PRO de la représentation BRO | | | | | | | | | | |
|---|---|---|---|---|---|---|---|---|---|---|
| Nom | $T_c$ ˚F | $P_c$ psi | $V_c$ ft³/lbm | $\omega$ | $\Omega b$ | Cv ft³/lbm | $\delta_{KK'}$ | V | I | H |
| V | -61.212 | 666.02 | 1.869 | -0.02043 | 0.079345 | -0.0686 | V | 0.0 | 0.058447 | 0.002065 |
| I | 368.292 | 581.21 | 4.389 | 0.31255 | 0.082877 | -0.0621 | I | # | 0.000000 | 0.001604 |
| H | 663.138 | 263.46 | 10.99 | 0.21233 | 0.065003 | 0.2664 | H | # | # | 0.000000 |

z) Dans le cas SPE3, on a choisi d'utiliser pour l'EOS_PRO une équation d'état du même type que celle utilisée pour la représentation de base. Ceci n'est pas obligatoire. Il est possible d'utiliser une équation d'état d'un autre type, dès lors que cette autre équation d'état utilise des paramètres de phase identifiables aux paramètres de phase utilisées par l'équation d'état de référence.

Plus généralement encore, on peut obtenir les paramètres nécessaires à la représentation BRO par diverses techniques dites de régression, inversion, optimisation, .. que l'on peut trouver dans une littérature abondante. On peut par exemple les rechercher par une ou plusieurs résolutions itératives de systèmes d'équations formés par des dérivées partielles, par rapport à des sous-ensembles ou à l'ensemble des paramètres à la représentation BRO, d'une fonction objectif du type de l'équation (**21**) ou d'un autre type.

On pourrait même chercher à utiliser un formalisme de ce type pour optimiser l'une ou plusieurs des masses molaires des constituants V, I, H, en la (les) faisant apparaître explicitement, - par remplacement dans la fonction objectif des compositions des phases par leurs expressions données par les équations (**8**) et (**9**) -, et en choisissant des observables sensibles à leur(s) valeur(s). Un telle démarche pourrait être envisageable si l'on se trouvait dans une situation, non rencontrée jusqu'à présent, où la ligne directrice suivie dans l'ensemble des paragraphes n) à w) conduisait à une détermination de paramètres de la représentation BRO ne permettant pas une reproduction fidèle des observables.

Enfin, pour des raisons connues en soi, on peut être amené à donner a priori des valeurs à certains des paramètres de l'équation EOS_PRO et n'effectuer alors des calculs d'estimation que pour un nombre réduit de paramètres.

aa) La première expression généralement attendue des résultats de simulation sont des résultats en volume. Les observables que l'on doit alors chercher à restituer sont les volumes molaires des phases en sortie de séparation. Pour le cas SPE3, on a utilisé deux corrélations distinctes, l'une pour le calcul des volumes molaires des phases gaz en sortie de séparation, l'autre pour le calcul molaire des phases huiles en sortie de séparation, les conditions de sortie de séparation étant les conditions standard usuelles de la profession à savoir une atmosphère et 15.56 ˚C (60 ˚F).

De nombreuses corrélations ou équations d'état sont disponibles pour estimer le volume molaire d'une phase gaz notamment dans des conditions proches des conditions atmosphériques. A titre indicatif, c'est la corrélation de P.M. Dranchuk et J.H. Abou-Kassem (présentée dans le papier : « Calculating Z Factors For Natural Gases Using Equations of State », JCPT, Juillet-Septembre 1975) qui a été utilisée pour le cas SPE3 où les paramètres utiles au calcul du volume molaire du gaz sont les pressions et températures critiques des constituants (V) et (I), paramètres qui ont été régressés de façon itérative de façon à reproduire les volumes molaires des phases gaz obtenus par les simulations PVT de séparation avec la représentation à 16 constituants.

La variation, en fonction de la fraction molaire du constituant I, du volume molaire des phases huile en des conditions proches des conditions atmosphériques a été trouvée, dans le cas SPE3, pratiquement linéaire, plus généralement, pour des cas non présentés ici, représentable par un polynôme de degré peu élevé (degré deux à trois). Pour le cas SPE3, c'est une dépendance linéaire qui a été adoptée dont les paramètres ont été obtenus par simple régression linéaire.

Les paramètres nécessaires aux calculs des volumes molaires en conditions de séparation ont été obtenus pour les deux chaînes de séparation CS_A et CS_B avec la même méthode.

*Equilibres thermodynamiques*

bb) Les calculs d'équilibre peuvent être effectués soit à partir de données de coefficients d'équilibre, soit à partir de l'écriture de l'égalité des fugacités de tous les constituants dans chaque phase, ces deux possibilités étant examinées respectivement ce qui suit.

cc) Selon la règle de Gibbs, pour le calcul des équilibres dans le réservoir, les coefficients d'équilibre des pseudo-composants (V), (I), (H) tels que calculés par le jeu d'équations (**10**) peuvent être introduits dans des tables comme fonctions de la pression, de la température, et d'un index de la composition dans la pseudo représentation. Ces données peuvent être utilisées ensuite comme données d'entrées dans un simulateur, le simulateur disposant en général de méthodes internes d'interpolation ou d'extrapolation pour estimer les coefficients d'équilibre en des points intermédiaires. On peut aussi, avant introduction des données dans un simulateur, ajouter des points aux tables en utilisant des méthodes, connues en soi, d'interpolation ou d'extrapolation ou utiliser, à la place des tables, des corrélations bâties pour restituer les coefficients d'équilibre.

Une dépendance des coefficients d'équilibre avec, tout à la fois la pression, la température et l'index de composition, n'est pas toujours nécessaire. Ainsi, dans nombre de cas d'application, la température du réservoir est considérée comme constante. Dans certains cas, on peut être amené à abandonner la dépendance par rapport à un index compositionnel. Ces cas où l'on réduirait le nombre des degrés de liberté des coefficients d'équilibre restent dans le domaine de l'utilisation de la méthode proposée.

dd) Pour le calcul des équilibres à l'issue d'une chaîne de séparation, les coefficients d'équilibre des pseudo-composants (V) et (H) sont respectivement infini et nul quelques soient les conditions de pression et de température en sortie de chaîne de séparation et quelque soit la composition du mélange soumis à la séparation. Si besoin est, dans un simulateur qui n'accepterait pas le concept de coefficients d'équilibre infini ou nul (ce concept étant souvent implémenté en forçant l'absence du constituant respectivement dans la phase huile et dans la phase gaz), on peut utiliser une valeur finie très grande et une valeur finie très faible, toutes deux positives, en lieu et place de coefficients d'équilibre infini ou nul. Pour les données relatives aux coefficients d'équilibre du constituant (I) nécessaires à un calcul d'équilibre, le jeu d'équations (**11**) indique une définition différente des coefficients d'équilibre du constituant (I) selon que l'on considère la séparation d'une phase 'gaz' issue du réservoir, ou la séparation d'une phase 'huile' issue du réservoir. Les entrées, en principe nécessaires aux calculs d'équilibre, sont donc, par phase, une table de coefficients d'équilibre du constituant (I) en fonction d'un index compositionnel de la phase soumise à la séparation, de la pression et de la température en sortie de la chaîne de séparation considérée. Les plages de variation des compositions des phases huile et gaz étant en général distinctes, il peut être pratique de 'fusionner' en une seule table les données utiles pour les phases huile et gaz, notamment si le simulateur n'accepte pas l'introduction de données distinctes pour chaque phase.

Ces données sont utilisables comme données d'entrées dans un simulateur, le simulateur disposant en général de méthodes internes d'interpolation ou d'extrapolation pour estimer les coefficients d'équilibre en des points intermédiaires. On peut aussi, avant introduction des données dans un simulateur, ajouter des points aux tables en utilisant des méthodes, connues en soi, d'interpolation ou d'extrapolation ou utiliser, à la place des tables, des corrélations bâties pour restituer les coefficients d'équilibre.

ee) Si l'équation d'état EOS_PRO est bâtie pour restituer les propriétés des phases, elle n'est pas forcément valide pour les calculs d'équilibre, c'est-à-dire pour permettre de restituer les compositions des phases de l'ensemble « E » obtenues dans le paragraphe g). Comme précédemment, on utilise ci-après l'équation d'état de Peng-Robinson comme support de présentation.

Pour cette équation d'état, les coefficients d'équilibre, d'après la condition d'égalité des fugacités de chaque constituant dans chaque phase à l'équilibre, s'obtiennent par:

$$(42) \quad \mathrm{Ln}(K_i) = D_0 + D_1\, b_i + \frac{P}{\sqrt{2}\, R^2\, T^2} \sum_{j=1}^{N} \left( \frac{L_G}{B_G}\, y_j - \frac{L_O}{B_O}\, x_j \right) a_{ij}(T)$$

avec les paramètres adimensionnels de phase définis selon les équations (**19**) et (**20**) et:

$$(43) \quad \left\{ \begin{array}{l} L_P = \mathrm{Ln}\!\left( \dfrac{Z_P + (1 + \sqrt{2})\, B_P}{Z_P + (1 - \sqrt{2})\, B_P} \right) \\[3ex] D_0 = \mathrm{Ln}\!\left( \dfrac{Z_G - B_G}{Z_O - B_O} \right) \\[3ex] D_1 = \dfrac{P}{R\,T}\left[ \dfrac{Z_O - 1}{B_O} - \dfrac{Z_G - 1}{B_G} + \dfrac{A_O\, L_O}{2\sqrt{2}\, B_O{}^2} - \dfrac{A_G\, L_G}{2\sqrt{2}\, B_G{}^2} \right] \end{array} \right.$$

et les facteurs de compressibilité des phases calculés à partir de l'équation (**18**).

Pour traiter un cas où la température de réservoir est constante, le problème est de déterminer les neuf paramètres indépendants formant un vecteur $\vec{p}$: $b_V$, $b_I$, $b_H$, $a_{VV}$, $a_{II}$, $a_{HH}$, $a_{VI}$, $a_{VH}$, $a_{IH}$, par exemple par minimisation d'une fonction objectif construite en utilisant comme observables $\overset{e}{_O K}$ les coefficients d'équilibre de l'ensemble « E » tels que calculés par les équations (**10**). La fonction objectif selon l'équation (**21**) peut alors être adaptée comme:

$$(44) \quad O(\vec{p}) = \sum_{e}^{e \in E} \sum_{K=V,I,H} \varpi_K^e \left[ \varphi_K^e(\vec{p}) - o_K^e \right]^2$$

où les fonctions $\varphi_K^e(\vec{p})$ sont les exponentielles des seconds membres des équations (**42**).

Alternativement, dans l'équation (**44**), on pourrait utiliser comme observables $o_K^e$ les logarithmes des coefficients d'équilibre de l'ensemble « E' » et comme fonctions $\varphi_K^e(\vec{p})$ sont les seconds membres des équations (**42**).

En suivant la démarche de K.E. Starling dans le papier: « A New Approach for Determining Equation-of-State Parameters Using Phase Equilibria Data » , SPE 1481, SPEJ, Décembre 1966, on peut choisir les poids $\varpi_K^e$ comme les inverses des valeurs des observables $o_K^e$.

La nullité des dérivées partielles de la fonction $O(\vec{p})$ par rapport aux neuf paramètres d'intérêt fournit un système de neuf équations non linéaires à neuf inconnues, résoluble uniquement de façon itérative, de nombreuses méthodes adaptées à la résolution des systèmes non linéaires étant discutées dans la littérature.

Un calcul itératif de ce type nécessite une première approximation du vecteur de paramètres. On a pu constater, notamment pour le cas SPE3, qu'il est opportun de tirer profit des paramètres obtenus pour l'EOS_PRO pour démarrer les calculs itératifs.

Plus généralement, notamment pour reproduire des coefficients d'équilibre à diverses température, on peut considérer un autre ensemble ou sous-ensemble de paramètres pour le vecteur $\vec{p}$ et d'autres techniques de recherche de paramètres.

ff) A titre d'illustration, pour le cas SPE3, ce sont les six paramètres $Pc_K$ et $\Omega b_K$ qui ont constitué le vecteur $\vec{p}$, les valeurs des autres paramètres étant reprises de l'équation EOS_PRO. La procédure utilisée dans le cas SPE3 est une procédure connue en soi en cours d'amélioration. L'ensemble des paramètres de l'EOS_EQ utilisés pour le cas SPE3 est donné dans le tableau 4 suivant :

| Tableau 4: Cas SPE3 - Paramètres de l'EOS_EQ de la représentation BRO | | | | | | | | |
|---|---|---|---|---|---|---|---|---|
| Nom | $T_c$ ˚F | $P_c$ psi | $\omega$ | $\Omega b$ | $\delta_{KK'}$ | V | I | H |
| V | -61.212 | 848.47 | -0.02043 | 0.089345 | V | 0.0 | 0.058447 | 0.002065 |
| I | 368.292 | 274.23 | 0.31255 | 0.070000 | I | # | 0.000000 | 0.001604 |
| H | 663.138 | 309.90 | 0.21233 | 0.065003 | H | # | # | 0.000000 |

gg) Dans le cas SPE3, les données introduites pour les calculs d'équilibre en surface pour chacune des chaînes de séparation CS_A et CS_B sont les coefficients d'équilibre du constituant (I) sous forme d'une table unique pour la séparation des phases huile et gaz, table obtenue comme décrit dans le paragraphe dd) avec pour seul abscisse l'index de la composition de la phase avant séparation.

hh) Les Fig. 2 à 5 comparent les résultats, sur les quinze années d'exploitation simulées du cas SPE3, d'une simulation compositionnelle à trois pseudo-composants BRO (résultats désignés par 'ternaire'), où la représentation compositionnelle a été obtenue par application de la méthode selon l'invention, à ceux d'une simulation compositionnelle détaillée à seize constituants de base (résultats désignés par 'référence').

La simulation compositionnelle de référence utilise une même équation d'état de Peng-Robinson pour les calculs d'équilibre et pour les calculs de propriétés de phase et pour toutes les zones thermodynamiques. La simulation BRO à trois constituants utilise, pour les calculs dans les mailles de réservoir, l'équation d'état (EOS_EQ) pour les calculs d'équilibre et l'équation d'état (EOS_PRO) pour les calculs de propriétés de phases. Pour les calculs en conditions de surface, la simulation BRO utilise des coefficients d'équilibre introduits sous forme de tables et des corrélations simples pour le calcul des volumes des phases.

Les résultats présentés Fig. 2, qui représentent l'évolution de pression en fond de puits producteur, et Fig. 5, qui représentent l'évolution de saturation en huile (ou condensat) dans la maille perforée la plus profonde à l'endroit du producteur, sont parmi les résultats les plus révélateurs du bon calcul des équilibres et des propriétés de phases dans les conditions in situ.

On peut constater que la modélisation BRO fournit une solution en pression pratiquement confondue avec la solution de référence (à 16 constituants) et une solution d'évolution de saturation en huile en bon accord avec la solution de référence sur tout le temps simulé. La maille utilisée en Fig. 5 est la même que celle utilisée dans le papier de référence de D.E. Kenyon et G. Alda Behie et dans le papier de W.H. Goldthorpe cité dans l'état de la technique. On peut constater que les résultats de la Fig. 5, jugés par comparaison avec les résultats obtenus par W.H. Goldthorpe avec une modélisation BO, montrent une bien meilleure qualité de reproduction de l'évolution de la saturation en huile dans le réservoir alors même que la procédure utilisée pour l'obtention des paramètres de l'EOS_EQ est améliorable.

ii) Les résultats présentés Fig. 3 et 4 de débit d'huile et de production cumulée d'huile sur toute la durée du temps simulé montre que la modélisation BRO fournit une solution très proche de la solution de référence.

La réduction de temps de calcul par la modélisation BRO est considérable, le facteur d'accélération étant de 46.

*Delumping*

jj) On représente tout d'abord le réservoir sous la forme d'un réseau de mailles (m) dont chacune constitue un volume élémentaire rempli de fluide(s) sous forme de une ou plusieurs phases, avec au moins une phase non aqueuse. Les phases non aqueuses restent dites phases hydrocarbure même si elles peuvent contenir certains composants autres que des hydrocarbures tel que l'azote, le dioxyde de carbone, l'anhydride sulfureux.

kk) On définit, pour chaque zone ou domaine thermodynamique, le fluide par une représentation détaillée à $N_{rb}$ composants et/ou pseudo-composants. Il est à noter que l'on peut traiter les cas qui requièrent l'usage de plusieurs représentations thermodynamiques, par exemple si l'on peut distinguer plusieurs chemins thermodynamiques locaux durant la modélisation (on peut être amené à distinguer des zones produites uniquement par dépressurisation et des zones soumises à une injection de gaz). Plusieurs zones ou domaines de variations des grandeurs thermodynamiques ou de composition, désignés souvent par les spécialistes comme zones thermodynamiques, pourront donc être définis et utilisés.

ll) Par zone thermodynamique pour laquelle on adopte une représentation groupée des fluides, on détermine une équation d'état EOS_PRO construite, préalablement à la simulation dynamique de réservoir avec la représentation groupée, pour reproduire les paramètres de phases, dans l'équation d'état de la représentation détaillée, des fluides hydrocarbure au cours de chemins thermodynamiques tenus pour représentatifs de ceux que vont suivre les fluides hydrocarbure pendant la simulation maillée.

mm) On réalise, de façon connue en soi, une simulation compositionnelle avec un nombre limité de constituants où les propriétés des phases sont calculées par une équation d'état EOS_PRO, ladite simulation permettant de calculer au moins dans chaque maille (m) et à des pas de temps consécutifs (t, t+1, etc.), une pression dans une phase hydrocarbure $(p_m^t)$, la température $(T_m^t)$ (si celle-ci varie), les débits molaires par phase d'injection ou de production $(S_{gm}^t)$ et $(S_{om}^t)$, pour chaque paire de mailles (m,h), les débits molaires des phases liquide $(u_{omh}^t)$ et vapeur $(u_{gmh}^t)$, la fraction vapeur $(\theta_m^t)$. On mémorise ces quantités, ainsi que les paramètres dans l'équation d'état EOS_PRO pour les phases hydrocarbure liquide et vapeur (typiquement les co-volumes et termes d'attraction) intervenant dans l'expression des coefficients d'équilibre. Pour éviter de les recalculer, on peut mémoriser également les facteurs de compressibilité des phases $(Z_{Om}^t)$ et $(Z_{Gm}^t)$ obtenus par résolution de l'équation d'état EOS_PRO. Pour une équation d'état de Peng-Robinson, en conservant les notations introduites par les équations (**16**), (**17**), (**26**) et (**27**), les paramètres de phase sont désignés par $(b_{Om}^t)$ et $(b_{Gm}^t)$ pour les co-volumes des phases, $(a_{Om}^t)$ et $(a_{Gm}^t)$, ou $(a_{1Om}^t)$, $(a_{1Gm}^t)$, $(a_{2Om}^t)$, $(a_{2Gm}^t)$ -, pour les termes d'attraction des phases.

Les différents paramètres utiles au calcul des coefficients d'équilibre, lorsque évoqués d'une façon générale, seront désignés ci-après par $(E_{\pi Pm}^t)$, l'indice P étant pour chaque phase huile et gaz, et l'indice $\pi$ un numéro de référence pour un paramètre particulier.

nn) On estime au pas de temps t+1 la fraction molaire de chaque constituant i dans la composition détaillée globale $(z_{im}^{t+1})$ du fluide hydrocarbure dans la maille (m) en connaissant les nombres de moles de chaque constituant i dans la représentation détaillée dans chaque phase au pas de temps t, respectivement $(No_{im}^{t})$ pour la phase huile et $(Ng_{im}^{t})$ pour la phase gaz à partir des équations ci-après:

$$(45) \quad No_m^t = \sum_i^{Nrb} No_{im}^t \quad ; \quad Ng_m^t = \sum_i^{Nrb} Ng_{im}^t \quad ; \quad N_m^t = No_m^t + Ng_m^t$$

$$(46) \quad x_{im}^t = \frac{No_{im}^t}{No_m^t} \quad ; \quad y_{im}^t = \frac{Ng_{im}^t}{Ng_m^t} \quad ; \quad z_{im}^t = \frac{No_{im}^t + Ng_{im}^t}{N_m^t}$$

$$(47) \quad z_{im}^{t+1} = \frac{z_{im}^t \, N_m^t - \Delta t \, (y_{im}^t \cdot S_{gm}^t + x_{im}^t \cdot S_{om}^t) - \Delta t \sum_{h \in J(m)} (y_{im}^t \cdot u_{gmh}^t + x_{im}^t \cdot u_{omh}^t)}{N_m^{t+1}}$$

$$(48) \quad N_m^{t+1} = N_m^t - \Delta t \, (S_{gm}^t + S_{om}^t) - \Delta t \sum_{h \in J(m)} (u_{gmh}^t + u_{omh}^t)$$

Les équations (45) et (46) permettent respectivement de calculer, au pas de temps (t) et dans la maille (m), les nombres de moles par phase hydrocarbure et global $N_m^t$ à partir de la connaissance des nombres de moles par constituant et par phase, et les fractions molaires des constituants par phase et globales.

L'équation (48) traduit le bilan molaire total hydrocarbure sur la maille (m), en tenant compte des échanges de matière, pendant l'intervalle de temps $\Delta t$, avec toutes les mailles (h) voisines de (m) qui forment l'ensemble J(m).

Dans l'équation (47), l'écriture des termes $(y_{ij'}^m)$ et $(x_{ij'}^m)$ , - dans lesquels m'=m pour un débit de la maille (m) vers la maille (h) ou dans le puits, et m'=h pour un débit de la maille (h) vers la maille (m), et m' correspondant au fluide injecté dans le cas des puits d'injection, S étant alors négatif -, suppose implicitement l'utilisation d'un schéma simple amont pour les flux compositionnels. Une écriture plus générale de ces termes est $(y_{imh}^t)$ et $(x_{imh}^t)$ où $x_{imh}^t$ et $y_{imh}^t$ décrivent les compositions des phases liquide et gaz, obtenues d'une façon connue en soi, s'écoulant entre les mailles (m) et (h).

oo) On détermine, au pas de temps (t+1) et dans chaque maille (m), les coefficients d'équilibre $(K_{im}^{t+1})$ du constituant i dans la représentation détaillée en utilisant une formulation où apparaissent explicitement les paramètres $E_{\pi P}$, en y introduisant les paramètres variables $(E_{\pi P \, m}^{t+1})$ issus de la simulation avec la représentation groupée, et, le cas échéant, en utilisant certaines des variables compositionnelles décrites dans le paragraphe précédent calculées au pas de temps (t). Ainsi, pour une équation d'état de Peng-Robinson, les coefficients d'équilibre donnés par l'équation (**42**) peuvent être réécrits sous la forme:

$$(49) \quad Ln(K_{i\,m}^{t+1}) = D_{0\,m}^{t+1} + D_{1\,m}^{t+1}\, b_i + D_{2\,m}^{t+1} \sqrt{a_i(T_m^{t+1})} + \frac{P_m^{t+1}}{\sqrt{2}\, R^2\, T_m^{t+1\,2}}\, I_{im}^{t+1}$$

avec:

$$(50) \quad \begin{cases} D_{0\,m}^{t+1} = Ln\left(\dfrac{Z_{G\,m}^{t+1} - B_{G\,m}^{t+1}}{Z_{O\,m}^{t+1} - B_{O\,m}^{t+1}}\right) \\[2em] L_{P\,m}^{t+1} = Ln\left(\dfrac{Z_{P\,m}^{t+1} + (1+\sqrt{2})\,B_{P\,m}^{t+1}}{Z_{P\,m}^{t+1} + (1-\sqrt{2})\,B_{P\,m}^{t+1}}\right) \quad P=O,G \\[2em] D_{1\,m}^{t+1} = \dfrac{P_m^{t+1}}{R\,T_m^{t+1}}\left[\dfrac{Z_{O\,m}^{t+1}-1}{B_{O\,m}^{t+1}} - \dfrac{Z_{G\,m}^{t+1}-1}{B_{G\,m}^{t+1}} + \dfrac{A_{O\,m}^{t+1}\,L_{O\,m}^{t+1}}{2\sqrt{2}\,B_{O\,m}^{t+1\,2}} - \dfrac{A_{G\,m}^{t+1}\,L_{G\,m}^{t+1}}{2\sqrt{2}\,B_{G\,m}^{t+1\,2}}\right] \\[2em] D_{2\,m}^{t+1} = \dfrac{P_m^{t+1}}{\sqrt{2}\,R^2\,T_m^{t+1\,2}}\left[\dfrac{L_{G\,m}^{t+1}}{B_{G\,m}^{t+1}}\,a_{1G\,m}^{t+1} - \dfrac{L_{O\,m}^{t+1}}{B_{O\,m}^{t+1}}\,a_{1O\,m}^{t+1}\right] \end{cases}$$

Dans les équations (**50**), on reconnaîtra les paramètres de phases adimensionnels B et A définis précédemment par les équations (**19**) et (**20**).

Dans l'équation (**49**), le terme $I_{im}^{t+1}$ représente la contribution des interactions binaires entre constituants et ce terme est nul lorsque les coefficients d'interactions binaires de la représentation détaillée sont tous nuls. Dans un tel cas, les coefficients d'équilibre peuvent être calculés directement à partir des paramètres $(E_{\pi P\,m}^{t+1})$ issus de l'équation d'état EOS_PRO de la simulation dynamique utilisant la représentation groupée.

Dans le cas contraire, une expression rigoureuse du terme $I_{im}^{t+1}$ est:

$$(51) \quad I_{im}^{t+1} = \frac{L_{O\,m}^{t+1}}{B_{O\,m}^{t+1}} \sum_{j=1}^{Nrb} \left[\sqrt{a_i(T_m^{t+1})}\,\sqrt{a_j(T_m^{t+1})}\,\delta_{ij}\,x_{j\,m}^{t+1}\right] - \frac{L_{G\,m}^{t+1}}{B_{G\,m}^{t+1}} \sum_{j=1}^{Nrb} \left[\sqrt{a_i(T_m^{t+1})}\,\sqrt{a_j(T_m^{t+1})}\,\delta_{ij}\,y_{j\,m}^{t+1}\right]$$

Ce terme fait donc intervenir les fractions molaires, dans la représentation détaillée, des constituants dans les phases hydrocarbure au pas de temps (t+1) $x_{i\,m}^{t+1}, y_{i\,m}^{t+1},$ non encore estimées. Pour éviter une résolution implicite lourde, on peut, dans l'équation (51), substituer les fractions molaires $x_{i\,m}^{t+1}, y_{i\,m}^{t+1}$ par les fractions molaires au pas de temps (t) issues des équations (**46**).

C'est avec une telle approximation, non limitative de réalisation, que les résultats de delumping :

- illustrés Fig. 8.1 à 8.16, Fig. 9.1 à 9.16 et récapitulés Fig.10 pour une opération de dépressurisation du fluide du cas SPE3,

- illustrés Fig. 11.1 à 11.16 et Fig. 12.1 à 12.16 et récapitulés Fig.13 pour une opération de vaporisation à une pression de 169 bar d'un condensat (le condensat obtenu à 169 bars lors de l'opération de dépressurisation précédente),

ont été obtenus.

Dans les Fig. 8.1 à 8.16 et 9.1 à 9.16 d'une part, Fig. 11.1 à 11.16 et 12.1 à 12.16 d'autre part, la teneur en chaque constituant dans la phase considérée est exprimée en pourcentage molaire de la phase, et, dans chaque figure, l'échelle est dilatée pour être adaptée à la variation de la teneur du constituant dans la phase.

L'abscisse sur les Fig. 11.1 à 11.16 et Fig. 12.1 à 12.16 est la fraction molaire de vapeur au cours de l'opération de vaporisation, qui croît de 0 (à l'état initial, seule la phase condensat, ou huile, est présente) à 1, lorsque tout le condensat est vaporisé.

La Fig. 10 et la Fig. 13 montrent que l'erreur absolue maximale obtenue sur la teneur en chaque constituant (toujours exprimée en pourcentage molaire) reste en moyenne inférieure à 0.1% durant les opérations de dépressurisation et de vaporisation.

On constate donc que les compositions issues de l'opération de delumping sont en bonne cohérence avec les compositions détaillées de référence des fluides, bien que l'approximation utilisée ne soit pas parmi les plus sophistiquées.

Une autre approximation possible, toujours non limitative de réalisation, est d'estimer les fractions molaires $x^{t+1}_{i\,m}$,

$y^{t+1}_{i\,m}$ à partir des coefficients d'équilibre $K^{t}_{im}$ calculables à partir des fractions molaires données par les équations (46) par:

$$(52) \quad \begin{cases} x^{t+1}_{im} = \dfrac{z^{t+1}_{im}\,(K^{t}_{im}-1)}{1+(K^{t}_{im}-1)\,\theta^{t+1}_{m}} \\[2em] y^{t+1}_{im} = \dfrac{k^{t}_{im}\,z^{t+1}_{im}\,(K^{t}_{im}-1)}{1+(K^{t}_{im}-1)\,\theta^{t+1}_{m}} \end{cases}$$

En général, les coefficients d'interactions binaires sont petits devant 1, et un calcul itératif, par exemple par substitutions successives, ne s'avère pas nécessaire, mais reste possible.

pp) On détermine, à chaque pas de temps (t+1), la fraction vaporisée $(\theta^{t+1}_{m})$ dans chaque maille (m), soit à partir des résultats de la simulation dynamique avec la représentation groupée soit, si une meilleure précision est recherchée, en résolvant l'équation de Rachford-Rice à partir des fractions molaires de chaque composant i dans la composition détaillée globale $(z^{t+1}_{im})$ du fluide hydrocarbure dans la maille (m) au pas de temps (t+1)

$$(53) \quad \sum_{i=1}^{Nrb} \dfrac{z^{t+1}_{im}\,(K^{t+1}_{im}-1)}{1+(K^{t+1}_{im}-1)\,\theta^{t+1}_{m}} = 0$$

Les nombres de moles par phase dans chaque maille à chaque pas de temps (t+1) sont donnés par:

$$(54) \quad No^{t+1}_m = N^{t+1}_m (1 - \theta^{t+1}_m) \quad ; \quad Ng^{t+1}_{im} = N^{t+1}_m \theta^{t+1}_m$$

qq) On estime la composition détaillée de chaque phase hydrocarbure, à chaque pas de temps (t+1) et dans chaque maille (m), en utilisant les relations suivantes :

$$(55) \quad x^{t+1}_{im} = \frac{z^{t+1}_{im} (K^{t+1}_{im} - 1)}{1 + (K^{t+1}_{im} - 1) \theta^{t+1}_m} \text{ pour la phase huile}$$

$$(56) \quad y^{t+1}_{im} = \frac{K^{t+1}_{im} z^{t+1}_{im} (K^{t+1}_{im} - 1)}{1 + (K^{t+1}_{im} - 1) \theta^{t+1}_m} \text{ pour la phase gaz}$$

rr) Enfin, on obtient les nombres de moles de chaque constituant i dans la représentation détaillée dans chaque phase au pas de temps t+1, respectivement $(No^{t+1}_{im})$ pour la phase huile et $(Ng^{t+1}_{im})$ par les équations (**46**) remaniées en:

$$(57) \quad No^{t+1}_{im} = No^{t+1}_m x^{t+1}_{im} \quad ; \quad Ng^{t+1}_{im} = Ng^{t+1}_m y^{t+1}_{im}$$

ss) Les paragraphes nn) à rr) détaillent le contenu des opérations pour passer du pas de temps t au pas de temps t+1, en supposant connus les nombres de moles de chaque constituant i dans la représentation détaillée dans chaque phase au pas de temps t dans chaque maille de la simulation avec la représentation groupée, respectivement $(No^t_{im})$ pour la phase huile et $(Ng^t_{im})$ pour la phase gaz. Pour démarrer les calculs, il suffit donc de connaître ces quantités à un instant ou pas de temps donné, qui peut être en particulier l'instant initial. Ceci peut être obtenu par divers moyens connus en soi, un moyen consistant en un calcul d'initialisation d'une simulation compositionnelle (calcul de l'état initial uniquement) utilisant la représentation compositionnelle détaillée, les entrées autres que compositionnelles étant les mêmes que celle de la simulation compositionnelle avec la représentation groupée.

Dès lors, on sait décrire l'évolution de la composition détaillée dans chaque maille au cours du procédé d'exploitation modélisé dans un modèle compositionnel utilisant une représentation groupée grâce à une mémoire des paramètres de l'équation d'état de la représentation détaillée conservée dans la méthode de calcul des propriétés de phases. Certaines étapes de calcul peuvent apparaître non nécessaires car redondantes. Certaines de ces redondances ont l'avantage de permettre de diminuer l'espace mémoire nécessaire au stockage des données, mais peuvent à l'évidence être évitées si les considérations d'espace mémoire ne sont pas prioritaires.

Dans le cas où l'EOS_PRO se révèle valide pour le calcul des coefficients d'équilibre (Ki,m et t+1), le delumping des résultats d'une simulation de réservoir effectué avec la méthode selon l'invention, peut s'effectuer par application de la méthode décrite dans la demande de brevet WO 99/42937 precitée.

*Remarques concernant la généralisation de l'approche*

tt) Les détails fournis dans la description de la méthode utilisent un formalisme molaire ; on aurait pu tout aussi bien utiliser un formalisme massique.

uu) On peut remarquer que le raisonnement basé sur l'hypothèse maîtresse « *M* » décrite dans le paragraphe d), appliqué à l'ensemble des constituants de la représentation de base, ensemble ci-après désigné par « **B** », dans les paragraphes précédents, aurait pu alternativement être appliqué à un sous-ensemble des constituants de la représentation de base, ci-après désigné par « R ». L'ensemble complémentaire de « R » dans « **B** » étant désigné par « S », les équations (**5**) seraient alors réécrites (avec **P=O,G**) comme:

$$(58) \begin{cases} MM_{PO}^e = x_{PI}^e\, MM_I + x_{PH}^e\, MM_H + \sum_i^{i\in S} x_{Pi}^e\, MM_i & ; & x_{PI}^e + x_{PH}^e = 1 - \sum_i^{i\in S} x_{Pi}^e \\[2em] MM_{PG}^e = y_{PV}^e\, MM_V + y_{PI}^e\, MM_I + \sum_i^{i\in S} y_{Pi}^e\, MM_i & ; & y_{PV}^e + y_{PI}^e = 1 - \sum_i^{i\in S} y_{Pi}^e \end{cases}$$

Une telle démarche peut être intéressante lorsque, pour le cas traité, il est besoin de garder explicites certains constituants de base dans la représentation groupée. Ainsi, dans un cas pratique où les conditions de réservoir ne permettraient pas de négliger la dissolution de certains constituants (tels le dioxyde de carbone) dans l'eau, ces constituants pourraient former l'ensemble « S ».

Les simples changements de variables indiqués ci-après permettent de ramener les équations (**5**) à la situation où l'on a considéré l'ensemble des constituants de la représentation de base:

$$(59) \begin{cases} MM_{PO}^e\Big|_R = \dfrac{MM_{PO}^e - \sum_i^{i\in S} x_{Pi}^e\, MM_i}{1 - \sum_i^{i\in S} x_{Pi}^e} & ; & MM_{PG}^e\Big|_R = \dfrac{MM_{PG}^e - \sum_i^{i\in S} y_{Pi}^e\, MM_i}{1 - \sum_i^{i\in S} y_{Pi}^e} \\[3em] x_{PI}^e\Big|_R = \dfrac{x_{PI}^e}{1 - \sum_i^{i\in S} x_{Pi}^e} \;;\; x_{PH}^e\Big|_R = \dfrac{x_{PH}^e}{1 - \sum_i^{i\in S} x_{Pi}^e} \;;\; y_{PV}^e\Big|_R = \dfrac{y_{PV}^e}{1 - \sum_i^{i\in S} y_{Pi}^e} \;;\; y_{PI}^e\Big|_R = \dfrac{y_{PI}^e}{1 - \sum_i^{i\in S} y_{Pi}^e} \end{cases}$$

Les équations (6) étant résolues avec les nouvelles variables, une transformation inverse de variables permet alors d'enchaîner les calculs des paragraphes g) et suivants.

La représentation groupée comporte alors les constituants (V), (I), (H) et les constituants de base de l'ensemble « S ». Dans chaque phase de l'ensemble « E », les fractions molaires des constituants de base formant l'ensemble « S » sont les mêmes dans la représentation groupée que dans la représentation de base.

vv) Les compositions des phases de l'ensemble « E » dans la représentation groupée étant ainsi obtenues, on peut suivre les grandes lignes des étapes décrites précédemment pour modéliser les propriétés de phases et les équilibres thermodynamiques. Dès lors qu'une équation d'état est recherchée pour la modélisation avec la représentation groupée des propriétés de phases et/ou des équilibres thermodynamiques, l'indication est de conserver pour les constituants de base formant l'ensemble « S » les paramètres de ces constituants dans l'équation d'état de la description détaillée. Les seuls paramètres à estimer restent alors les paramètres des constituants (V), (I), (H) dans l'équation d'état, et le cas échéant les paramètres d'interactions binaires de ces constituants avec les constituants de l'ensemble « S ». Lorsque ces derniers sont posés comme nuls, on peut suivre la ligne directrice suivie dans l'ensemble des paragraphes n) à w) qui est de rechercher les paramètres d'équation d'état EOS_PRO utiles aux calculs, dans les conditions de réservoir, de densités des phases (via les facteurs de compressibilité) et de viscosités par des résolutions simples (non itératives) de systèmes d'équations linéaires.

**Revendications**

1. Méthode de pseudoïsation pour estimer les propriétés ou le comportement des phases hydrocarbure liquide et/ou vapeur à partir de données relatives à un ensemble de référence constitué de mélanges hydrocarbonés dans une série d'états thermodynamiques découlant de conditions d'exploitation définies d'un gisement souterrain d'hydrocarbures, **caractérisée en ce que** :

   - on regroupe chacun des dits mélanges hydrocarbonés en au moins trois constituants (V, I, H), aucun de ces constituants ne correspondant à une sélection particulière de composants ou pseudo-composants de base qui serviraient à une description compositionnelle détaillée des fluides, en considérant que les phases gaz issues de la séparation en conditions de surface de chacun des mélanges hydrocarbonés sont des mélanges dont le troisième constituant (H) est exclu, et que les phases huile issues de la séparation en conditions de surface de chacun des mélanges hydrocarbonés sont des mélanges dont le premier constituant (V) est exclu ;
   - on détermine par bilan de matière les compositions des produits de séparation comprenant, pour les produits gazeux, au moins le premier et le deuxième constituant (V, I) en proportions variables, et pour les produits liquides, au moins le deuxième et le troisième constituant (I, H) en proportions variables ; et
   - on détermine la composition à au moins trois constituants de chaque mélange hydrocarboné de l'ensemble de référence par combinaison des produits de sa séparation au prorata des quantités de chaque produit de séparation.

2. Méthode selon la revendication 1, dans laquelle on regroupe chacun des mélanges hydrocarbonés en seulement trois constituants (V, I, H), les phases gaz issues de la dite séparation étant des mélanges en proportions variables du premier constituant (V) et du deuxième constituant (I), les phases huile issues de la dite séparation étant des mélanges en proportions variables du deuxième constituant (I) et du troisième constituant (H), et on détermine la composition à trois constituants.

3. Méthode selon la revendication 1 ou 2, dans laquelle les conditions de surface sont celles rencontrées ou prévues lors de l'exploitation du gisement.

4. Méthode selon la revendication 1 ou 2, dans laquelle les conditions de surface sont différentes de celles rencontrées ou prévues lors de l'exploitation du gisement

5. Méthode selon la revendication 1 ou 2, dans laquelle le bilan de matière est un bilan massique et dans laquelle on attribue à chacun des trois constituants (V, I, H) une masse molaire après une analyse quantitative des masses molaires des produits de séparation de l'ensemble de référence.

6. Méthode selon la revendication 1 ou 2, dans laquelle on définit les données nécessaires aux calculs d'équilibre et à la modélisation des propriétés de phases dans la représentation groupée, en utilisant les compositions des phases dans la représentation groupée et des données connues, ou estimées, a priori portant au moins sur la densité et la viscosité de phases huile et gaz en équilibre appartenant à l'ensemble de référence.

7. Méthode selon la revendication 6, dans laquelle, lorsque les dites données incluent des données compositionnelles détaillées des phases préalablement représentées par une équation d'état, on définit les paramètres d'une première équation d'état de la représentation groupée, utile à la modélisation des propriétés de phases, en utilisant ces données compositionnelles.

8. Méthode selon la revendication 6, dans laquelle on ajuste les paramètres d'une seconde équation d'état de la représentation groupée, utile aux calculs d'équilibre, pour reproduire les coefficients d'équilibre de la représentation groupée.

9. Méthode selon la revendication 8, dans laquelle on utilise, dans la procédure d'ajustement pour définir les paramètres de la seconde équation d'état de la représentation groupée utile aux calculs d'équilibre, les paramètres par constituant de la représentation groupée dans la première équation d'état.

10. Méthode selon l'une des revendications précédentes, dans laquelle on détermine les coefficients d'équilibre des fluides dans une représentation compositionnelle détaillée, à partir de variables et/ou paramètres intervenant dans le calcul des propriétés de phases, dès lors que les paramètres utiles aux calculs des propriétés de phases dans la représentation groupée ont été estimés de façon à reproduire les paramètres des phases dans l'équation d'état de la description compositionnelle détaillée.

**11.** Méthode selon l'une des revendications précédentes, comportant un éclatement ou delumping pour prédire en fonction du temps et dans au moins une zone thermodynamique, une composition détaillée d'un fluide contenu dans un gisement d'hydrocarbures ou produit par au moins un puits.

**12.** Méthode selon la revendication 11 dans laquelle :

- on représente le réservoir sous la forme d'un réseau de mailles (m) dont chacune constitue un volume élémentaire rempli de fluides sous forme de une ou plusieurs phases, avec au moins une phase non aqueuse ;
- on définit, pour chaque zone ou domaine thermodynamique, les fluides par une représentation détaillée de base, de façon à déterminer la quantité de chaque constituant de base (i) dans chaque phase hydrocarbure dans chaque maille (m) à l'instant défini comme initial pour le calcul de delumping ;
- par zone thermodynamique pour laquelle on adopte une représentation groupée des fluides, on détermine une équation d'état construite, préalablement à la simulation dynamique de réservoir avec la représentation groupée, pour reproduire les paramètres de phases, dans l'équation d'état de la représentation détaillée, des fluides hydrocarbonés au cours de chemins thermodynamiques tenus pour représentatifs de ceux que vont suivre les fluides hydrocarbonés pendant la simulation maillée ;
- on réalise à un pas de temps t une simulation compositionnelle avec un nombre limité de constituants où les propriétés des phases sont calculées par une équation d'état, ladite simulation permettant de calculer au moins dans chaque maille (m) et à des pas de temps consécutifs, une pression pour une phase hydrocarbure, la température lorsque celle-ci varie, les débits des phases entre mailles et au niveau des perforations de production et d'injection, et les valeurs de paramètres et/ou propriétés de phase intervenant dans l'expression formelle des coefficients d'équilibre de la représentation détaillée, et on mémorise ces diverses quantités ;
- on estime au pas de temps suivant (t+1) la fraction molaire de chaque constituant i dans la composition détaillée globale du fluide hydrocarboné dans la maille (m) par bilan matière sur la maille (m) ;
- on détermine en utilisant les quantités mémorisées, au même pas de temps (t+1) et dans chaque maille (m), les coefficients d'équilibre de chaque constituant (i) dans la représentation détaillée ;
- on détermine, au même pas de temps (t+1), la fraction vaporisée dans chaque maille (m) ; et
- on estime la composition détaillée de chaque phase hydrocarbonée, au même pas de temps (t+1) et dans chaque maille (m).

**13.** Méthode selon l'une des revendications précédentes, où différentes étapes sont traduites de façon à produire des résultats intermédiaires ou finaux utilisables dans des équations exprimées selon un formalisme molaire ou massique.

**Claims**

**1.** A lumping method for estimating the properties or the behaviour of liquid and/or vapour hydrocarbon phases from data relative to a reference set consisting of hydrocarbon mixtures in a series of thermodynamic states resulting from determined conditions of production of an underground hydrocarbon reservoirs, **characterized in that** it comprises :

- grouping each one of said hydrocarbon mixtures into at least three constituents (V, I, H), none of these constituents corresponding to a particular selection of base components or pseudo-components that would be used for a detailed compositional description of the fluids, considering that the gas phases resulting from the separation under surface conditions of each one of the hydrocarbon mixtures are mixtures from which third constituent (H) is excluded, and that the oil phases resulting from the separation under surface conditions of each one of the hydrocarbon mixtures are mixtures from which first constituent (V) is excluded,
- determining by material balance the compositions of the separation products comprising, for the gaseous products, at least the first and the second constituent (V, I) in variable proportions and, for the liquid products, at least the second and the third constituent (I, H) in variable proportions, and
- determining the at least three-constituent composition of each hydrocarbon mixture of the reference set by combination of the products of the separation thereof in proportion to the amounts of each separation product.

**2.** A method as claimed in claim 1, wherein each one of the hydrocarbon mixtures is grouped into only three constituents (V, I, H), the gas phases resulting from said separation being mixtures in variable proportions of first constituent (V) and of second constituent (I), the oil phases resulting from said separation are mixtures in variable proportions of second constituent (I) and of third constituent (H), and the three-constituent composition is determined.

3. A method as claimed in claim 1 or 2, wherein the surface conditions are the conditions encountered or expected during reservoir production.

4. A method as claimed in claim 1 or 2, wherein the surface conditions are different from the conditions encountered or expected during reservoir production.

5. A method as claimed in claim 1 or 2, wherein the material balance is a mass balance and a molar mass is assigned to each one of the three constituents (V, I, H) after quantitative analysis of the molar masses of the separation products of the reference set.

6. A method as claimed in claim 1 or 2, wherein the data necessary for equilibrium calculation and for modelling the phase properties in the lumped representation are defined using the compositions of the phases in the lumped representation and known or estimated a priori data relative to at least the density and the viscosity of the oil and gas phases at equilibrium belonging to the reference set.

7. A method as claimed in claim 6 wherein, when said data includes detailed compositional data of the phases previously represented by a state equation, the parameters of a first state equation of the lumped representation, used for modelling the phase properties, are defined using this compositional data.

8. A method as claimed in claim 6, wherein the parameters of a second state equation of the lumped representation, useful for equilibrium calculations, are adjusted in order to reproduce the equilibrium coefficients of the lumped representation.

9. A method as claimed in claim 8, wherein the parameters per constituent of the lumped representation in the first state equation are used in the adjustment procedure for defining the parameters of the second state equation of the lumped representation useful for equilibrium calculations.

10. A method as claimed in any one of the previous claims, wherein the equilibrium coefficients of the fluids are determined in a detailed compositional representation, from variables and/or parameters involved in the calculation of the phase properties, from the moment that the parameters useful for calculation of the phase properties in the lumped representation have been estimated so as to reproduce the parameters of the phases in the state equation of the detailed compositional description.

11. A method as claimed in any one of the previous claims, comprising delumping for predicting as a function of time, and in at least one thermodynamic zone, a detailed composition of a fluid contained in a hydrocarbon reservoir or produced by at least one well.

12. A method as claimed in claim 11, comprising :

- representing the reservoir in form of a network of grid cells (m) wherein each one forms an elementary volume filled with fluids in form of one or more phases, with at least one nonaqueous phase,
- defining, for each thermodynamic zone or range, the fluids by a detailed base representation, so as to determine the amount of each base constituent (i) in each hydrocarbon phase in each grid cell (m) at the time defined as initial for the delumping calculation,
- per thermodynamic zone for which a lumped representation of the fluids is selected, determining a state equation constructed prior to dynamic reservoir simulation with the lumped representation, to reproduce the phase parameters, in the state equation of the detailed representation, of the hydrocarbon fluids along thermodynamic paths considered to be representative of those that will be followed by the hydrocarbon fluids during the gridded simulation,
- carrying out, at a time interval t, a compositional simulation with a limited number of constituents wherein the phase properties are calculated by a state equation, said simulation allowing to calculate at least in each grid cell (m) and at consecutive time intervals a pressure for a hydrocarbon phase, the temperature when it varies, the flow rates of the phases between grid cells and at the production and injection perforations, and the values of parameters and/or phase properties involved in the formal expression of the equilibrium coefficients of the detailed representation, and storing these various quantities,

- estimating at the next time interval (t+1) the molar fraction of each constituent i in the global detailed composition of the hydrocarbon fluid in grid cell (m) by material balance on grid cell (m),
- determining, using the quantities stored, at the same time interval (t+1) and in each grid cell (m), the equilibrium coefficients of each constituent (i) in the detailed representation,
- determining, in the same time interval (t+1), the vaporized fraction in each grid cell (m), and
- estimating the detailed composition of each hydrocarbon phase, at the same time interval (t+1) and in each grid cell (m).

**13.** A method as claimed in any one of the previous claims, wherein various stages are translated so as to produce intermediate or final results usable in equations expressed according to a molar or mass formalism.

**Patentansprüche**

**1.** Verfahren zur Pseudoisierung, um die Eigenschaften oder das Verhalten von flüssigen und/oder dampfförmigen Kohlenwasserstoffphasen zu ermitteln, ausgehend von Daten, die sich auf eine Referenzmenge beziehen, die aus Kohlenwasserstoffgemischen in einer Reihe von thermodynamischen Zuständen besteht, die sich aus definierten Ausbeutungsbedingungen einer unterirdischen Kohlenwasserstofflagerstätte ergeben, **dadurch gekennzeichnet, dass**:

- jedes der Kohlenwasserstoffgemische in mindestens drei Bestandteile (V, I, H) eingeteilt wird, wobei keiner dieser Bestandteile einer bestimmten Auswahl an Basisbestandteilen oder Pseudobasisbestandteilen entspricht, die einer detaillierten Beschreibung der Zusammensetzung der Fluide dienen würden, indem angenommen wird, dass die Gasphasen, die aus der Trennung jedes der Kohlenwasserstoffgemische unter Oberflächenbedingungen stammen, Gemische sind, deren dritter Bestandteil (H) ausgeschlossen ist, und dass die Ölphasen, die aus der Trennung jedes der Kohlenwasserstoffgemische unter Oberflächenbedingungen stammen, Gemische sind, deren erster Bestandteil (V) ausgeschlossen ist;
- mittels Materialbilanz die Bestandteile der Trennungsprodukte bestimmt werden, die für die gasförmigen Produkte mindestens den ersten und den zweiten Bestandteil (V, I) in variablen Anteilen, und für die flüssigen Produkte mindestens den zweiten und den dritten Bestandteil (I, H) in variablen Anteilen umfassen;
- und die Zusammensetzung aus mindestens drei Bestandteilen jedes der Kohlenwasserstoffgemische der Referenzmenge durch Kombination der Produkte aus ihrer Trennung im Verhältnis zu den Mengen jedes Trennungsprodukts bestimmt wird.

**2.** Verfahren nach Anspruch 1, wobei jedes der Kohlenwasserstoffgemische in nur drei Bestandteile (V, I, H) eingeteilt wird, wobei die Gasphasen, die aus der Trennung stammen, Gemische mit variablen Anteilen des ersten Bestandteils (V) und des zweiten Bestandteils (I) sind, wobei die Ölphasen, die aus der Trennung stammen, Gemische in variablen Anteilen des zweiten Bestandteils (I) und des dritten Bestandteils (H) sind, und die Zusammensetzung aus drei Bestandteilen bestimmt wird.

**3.** Verfahren nach Anspruch 1 oder 2, wobei die Oberflächenbedingungen jene sind, die bei der Ausbeutung der Lagerstätte angetroffen oder erwartet werden.

**4.** Verfahren nach Anspruch 1 oder 2, wobei sich die Oberflächenbedingungen von jenen unterscheiden, die bei der Ausbeutung der Lagerstätte angetroffen oder erwartet werden.

**5.** Verfahren nach Anspruch 1 oder 2, wobei die Materialbilanz eine Massenbilanz ist und wobei jedem der drei Bestandteile (V, 1, H) nach einer quantitativen Analyse der Molmassen der Trennungsprodukte der Referenzmenge eine Molmasse zugeordnet wird.

**6.** Verfahren nach Anspruch 1 oder 2, wobei die Daten definiert werden, die für die Gleichgewichtsberechnungen und die Modellierung der Phaseneigenschaften in der gruppierten Darstellung erforderlich sind, indem die Zusammensetzungen der Phasen in der gruppierten Darstellung und bekannte oder ermittelte Daten verwendet werden, die a priori mindestens die Dichte und die Viskosität der Öl- und Gasphasen im Gleichgewicht betreffen, die zur Referenzmenge gehören.

**7.** Verfahren nach Anspruch 6, wobei, wenn die Daten detaillierte Daten zur Zusammensetzung der zuvor durch eine Zustandsgleichung dargestellten Phasen beinhalten, die Parameter einer ersten Zustandsgleichung der gruppierten Darstellung definiert werden, die für die Modellierung der Phaseneigenschaften nützlich ist, indem diese Daten der Zusammensetzung verwendet werden.

8.  Verfahren nach Anspruch 6, wobei die Parameter einer zweiten Zustandsgleichung der gruppierten Darstellung , die für die Gleichgewichtsberechnungen nützlich sind, angepasst werden, um die Gleichgewichtskoeffizienten der gruppierten Darstellung zu reproduzieren.

9.  Verfahren nach Anspruch 8, wobei in dem Anpassungsvorgang zur Definition der Parameter der zweiten Zustandsgleichung der gruppierten Darstellung, die für die Gleichgewichtsberechnungen nützlich sind, die Parameter je Bestandteil der gruppierten Darstellung in der ersten Zustandsgleichung verwendet werden.

10. Verfahren nach einem der vorhergehenden Ansprüche, wobei die Gleichgewichtskoeffizienten der Fluide in einer detaillierten Darstellung der Zusammensetzung ausgehend von Variablen und/oder Parametern bestimmt werden, die an der Berechnung der Phaseneigenschaften beteiligt sind, sobald die Parameter, die für die Berechnungen der Phaseneigenschaften in der gruppierten Darstellung nützlich sind, so bestimmt wurden, dass sie die Parameter der Phasen in der Zustandsgleichung der detaillierten Beschreibung der Zusammensetzung reproduzieren.

11. Verfahren nach einem der vorhergehenden Ansprüche, das eine Deagglomeration oder Delumping umfasst, um in Abhängigkeit von der Zeit und in mindestens einer thermodynamischen Zone eine detaillierte Zusammensetzung eines Fluids vorherzusagen, das in einer Kohlenwasserstofflagerstätte enthalten ist oder von mindestens einem Bohrloch produziert wird.

12. Verfahren nach Anspruch 11, wobei:

- das Reservoir in Form eines Netzes aus Gitterzellen (m) dargestellt wird, wovon jede einzelne ein Elementarvolumen bildet, das mit Fluiden in Form von einer oder mehreren Phasen gefüllt ist, mit mindestens einer nicht wässrigen Phase;
- für jede(s) thermodynamische Zone oder Gebiet, die Fluide durch eine detaillierte Basisdarstellung definiert werden, um die Menge jedes Basisbestandteils (i) in jeder Kohlenwasserstoffphase in jeder Gitterzelle (m) zu dem Zeitpunkt zu bestimmen, der als Ausgangspunkt für die Delumping-Berechnung definiert wurde;
- je thermodynamischer Zone, für die eine gruppierte Darstellung der Fluide eingeführt wird, eine konstruierte Zustandsgleichung vor der dynamischen Simulation des Reservoirs mit der gruppierten Darstellung bestimmt wird, um die Phasenparameter in der Zustandsgleichung der detaillierten Darstellung von Kohlenwasserstofffluiden im Verlauf von thermodynamischen Pfaden zu reproduzieren, die als repräsentativ für jene angenommen werden, denen die Kohlenwasserstofffluide während der Gitterzellensimulation folgen werden;
- mit einem Zeitintervall t eine Simulation der Zusammensetzung mit einer begrenzten Anzahl von Bestandteilen ausgeführt wird, wobei die Eigenschaften der Phasen durch eine Zustandsgleichung berechnet werden, wobei es die Simulation ermöglicht, mindestens in jeder Gitterzelle (m) und in aufeinander folgenden Zeitintervallen einen Druck für eine Kohlenwasserstoffphase, die Temperatur, wenn diese variiert, die Durchflussmengen der Phasen zwischen Gitterzellen und an den Produktions- und Injektionsperforationen und die Werte der Phasenparameter und/oder - eigenschaften, die an dem formalen Ausdruck der Gleichgewichtskoeffizienten der detaillierten Darstellung beteiligt sind, zu berechnen, und diese verschiedenen Mengen zu speichern;
- mit folgendem Zeitintervall (t+1) die Molfraktion jedes Bestandteils i in der detaillierten Gesamtzusammensetzung des Kohlenwasserstofffluids in der Gitterzelle (m) mittels Materialbilanz auf der Gitterzelle (m) ermittelt wird;
- unter Verwendung der gespeicherten Mengen im gleichen Zeitintervall (t+1) und in jeder Gitterzelle (m) die Gleichgewichtskoeffizienten jedes Bestandteils (i) in der detaillierten Darstellung bestimmt werden;
- im gleichen Zeitintervall (t+1) die verdampfte Fraktion in jeder Gitterzelle (m) bestimmt wird; und
- die detaillierte Zusammensetzung jeder Kohlenwasserstoffphase im gleichen Zeitintervall (t+1) und in jeder Gitterzelle (m) ermittelt wird.

13. Verfahren nach einem der vorhergehenden Ansprüche, wobei verschiedene Schritte so übersetzt werden, dass sie Zwischen- oder Endergebnisse produzieren, die in Gleichungen verwendbar sind, die gemäß einem Mol- oder Massenformalismus ausgedrückt werden.

# FIG.1

| "Réservoir" | | "Surface" | |
|---|---|---|---|

Conditions « in situ »: $T^e$, $P^e$

Masses molaires des phases | Fractions molaires des phases

G → Séparation →

G    $MM_{GG}^e$    $\theta_G^e$

O    $MM_{GO}^e$    $1 - \theta_G^e$

O → Séparation →

G    $MM_{OG}^e$    $\theta_O^e$

O    $MM_{OO}^e$    $1 - \theta_O^e$

**FIG.2**

**FIG.3**

**FIG.4**

**FIG.5**

**FIG.6**

**FIG.7**

## FIG.8-1

**Fraction molaire de C1 dans le gaz**

◊ référence
— delumping

## FIG.9-1

**Fraction molaire de C1 dans l'huile**

◊ référence
— delumping

## FIG.8-2

**Fraction molaire de N2 dans le gaz**

◊ référence
— delumping

## FIG.9-2

**Fraction molaire de N2 dans l'huile**

◊ référence
— delumping

## FIG.8-3

**Fraction molaire de C2 dans le gaz**

◊ référence
— delumping

## FIG.9-3

**Fraction molaire de C2 dans l'huile**

◊ référence
— delumping

## FIG.8-4

**Fraction molaire de CO2 dans le gaz**

## FIG.9-4

**Fraction molaire de CO2 dans l'huile**

## FIG.8-5

**Fraction molaire de C3 dans le gaz**

## FIG.9-5

**Fraction molaire de C3 dans l'huile**

## FIG.8-6

**Fraction molaire de IC4 dans le gaz**

## FIG.9-6

**Fraction molaire de IC4 dans l'huile**

**FIG.8-7**

Fraction molaire de C4 dans le gaz

**FIG.9-7**

Fraction molaire de C4 dans l'huile

**FIG.8-8**

Fraction molaire de IC5 dans le gaz

**FIG.9-8**

Fraction molaire de IC5 dans l'huile

**FIG.8-9**

Fraction molaire de C5 dans le gaz

**FIG.9-9**

Fraction molaire de C5 dans l'huile

## FIG.8-10

**Fraction molaire de C6 dans le gaz**

## FIG.9-10

**Fraction molaire de C6 dans l'huile**

## FIG.8-11

**Fraction molaire de C7 dans le gaz**

## FIG.9-11

**Fraction molaire de C7 dans l'huile**

## FIG.8-12

**Fraction molaire de C8 dans le gaz**

## FIG.9-12

**Fraction molaire de C8 dans l'huile**

## FIG.8-13

**Fraction molaire de C9 dans le gaz**

## FIG.9-13

**Fraction molaire de C9 dans l'huile**

## FIG.8-14

**Fraction molaire de C10 dans le gaz**

## FIG.9-14

**Fraction molaire de C10 dans l'huile**

## FIG.8-15

**Fraction molaire de C11 dans le gaz**

## FIG.9-15

**Fraction molaire de C11 dans l'huile**

**FIG.8-16**

**Fraction molaire de C12P dans le gaz**

◇ référence
—— delumping

(%)

Pression (bar)

**FIG.9-16**

**Fraction molaire de C12P dans l'huile**

◇ référence
—— delumping

(%)

Pression (bar)

**FIG.10**

**Maximum de l'erreur absolue sur les fractions molaires des phases huile et gaz exprimées en %**

(%)

◇ huile
+ gaz

Fraction molaire par constituant dans le fluide initial (%)

## FIG.11-1

**Fraction molaire de C1 dans le gaz**

## FIG.12-1

**Fraction molaire de C1 dans l'huile**

## FIG.11-2

**Fraction molaire de N2 dans le gaz**

## FIG.12-2

**Fraction molaire de N2 dans l'huile**

## FIG.11-3

**Fraction molaire de C2 dans le gaz**

## FIG.12-3

**Fraction molaire de C2 dans l'huile**

47

## FIG.11-4

**Fraction molaire de CO2 dans le gaz**

## FIG.12-4

**Fraction molaire de CO2 dans l'huile**

## FIG.11-5

**Fraction molaire de C3 dans le gaz**

## FIG.12-5

**Fraction molaire de C3 dans l'huile**

## FIG.11-6

**Fraction molaire de IC4 dans le gaz**

## FIG.12-6

**Fraction molaire de IC4 dans l'huile**

## FIG.11-7

**Fraction molaire de C4 dans le gaz**

## FIG.12-7

**Fraction molaire de C4 dans l'huile**

## FIG.11-8

**Fraction molaire de IC5 dans le gaz**

## FIG.12-8

**Fraction molaire de IC5 dans l'huile**

## FIG.11-9

**Fraction molaire de C5 dans le gaz**

## FIG.12-9

**Fraction molaire de C5 dans l'huile**

## FIG.11-10

**Fraction molaire de C6 dans le gaz**

## FIG.12-10

**Fraction molaire de C6 dans l'huile**

## FIG.11-11

**Fraction molaire de C7 dans le gaz**

## FIG.12-11

**Fraction molaire de C7 dans l'huile**

## FIG.11-12

**Fraction molaire de C8 dans le gaz**

## FIG.12-12

**Fraction molaire de C8 dans l'huile**

## FIG.11-13

**Fraction molaire de C9 dans le gaz**

## FIG.12-13

**Fraction molaire de C9 dans l'huile**

## FIG.11-14

**Fraction molaire de C10 dans le gaz**

## FIG.12-14

**Fraction molaire de C10 dans l'huile**

## FIG.11-15

**Fraction molaire de C11 dans le gaz**

## FIG.12-15

**Fraction molaire de C11 dans l'huile**

## FIG.11-16

**Fraction molaire de C12P dans le gaz**

◇ référence
— delumping

(%)

Fraction molaire de vapeur

## FIG.12-16

**Fraction molaire de C12P dans l'huile**

◇ référence
— delumping

(%)

Fraction molaire de vapeur

## FIG.13

**Maximum de l'erreur absolue sur les fractions molaires des phases huile et gaz exprimées en %**

(%)

◇ huile
+ gaz

Fraction molaire par constituant dans le fluide initial (%)

**EP 1 462 605 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0037898 A
- WO 9942937 A
- FR 0009008
- FR 2811430

**Littérature non-brevet citée dans la description**

- **K. Liu.** Reduce the Number of Components for Compositional Reservoir Simulation. *SPE Reservoir Simulation Symposium,* 11 Février 2001
- **D.E. Kenyon ; G. Alda Behie.** Third SPE Comparative Solution Project: Gas Cycling of Retrograde Condensate Reservoirs. *SPE 12278, Journal of Petroleum Technology,* Août 1987
- **C. Leibovici ; J. Barker.** A Method for Delumping the Results of a Compositional Reservoir Simulation. *SPE Annual Technical Conference and Exhibition New-Orleans,* 27 Septembre 1998
- **C.F. Leibovici ; E.H. Stenby ; K. Knudsen.** A Consistent Procedure for Pseudo-Component Delumping. *Fluid Phase Equilibria,* 1996, vol. 117, 225-232
- **W.H. Goldthorpe.** Simulation of Gas Injection Processes in Gas-Condensate Reservoirs Using a Binary Pseudo-Component Representation. *SPE Asia-Pacific Conference,* 13 Septembre 1989
- **Lohrenz, John ; Bray, B.G. ; Clark, C.R.** Calculating Viscosities of Reservoir Fluids from Their Compositions. *Journal of Petroleum Technology,* 1964, 1171-1176
- **P.M. Dranchuk ; J.H. Abou-Kassem.** Calculating Z Factors For Natural Gases Using Equations of State. *JCPT,* Juillet 1975